# EUROPEAN PATENT SPECIFICATION

(11) **EP 0 933 376 B1**
(45) Date of publication and mention of the grant of the patent: **20.08.2003**
(21) Application number: 98124497.3
(22) Date of filing: 29.12.1998
(51) Int. Cl.: C07F 7/08, C07D 251/54, A61K 7/42

(54) **Silanyl-triazines as light screening compositions**
Silanyltriazine als Lichtschutzmittel
Silanyltriazines comme compositions de protection solaire

(30) Priority: 02.01.1998 EP 98100008; 24.11.1998 EP 98122321
(43) Date of publication of application: 04.08.1999
(73) Proprietor: F. HOFFMANN-LA ROCHE AG, 4070 Basel (CH)
(72) Inventor: Huber, Ulrich, 8703 Erlenbach (CH)
(74) Representative: Keller, Günter, Dr.

(56) References cited:
- EP-A- 0 693 483
- EP-A- 0 751 170
- US-A- 4 617 390

## Description

The present invention relates to new s-triazines, a process for their manufacture and the use thereof as UV-B filters in light screening compositions, especially for the preparation of a cosmetic composition useful for protecting human skin from sunlight radiation.

Many sunscreen agents have been described and developed in the past proposing 1,3,5 triazines as UV-B stabilizers. An example for such a triazine-compound is 4,4',4"-(1,3,5-triazine-2,4,6-triyltriimino)-tris-benzoic acid-tris(2-ethylhexyl ester) sold under trade name UVINUL T-150.

In the German Patent Publication DE-OS 3 206 3987 which is an equivalent of US Patent 4 617 390, s-triazine derivatives are disclosed which highly absorb in the UV-B region and are obtained by reacting trichlorotriazine with p-aminobenzoic acid esters. However, these compounds are very poorly soluble in the solvents commonly used in the formulations of sunscreen agents, thus limiting their use as ingredient of emulsions and cosmetic formulations, particularly when an increased dosage of the sunscreen agent is needed. In addition those filters tend to crystallise on the skin leaving a sandy skin feeling and reducing the sun protection factor (SPF) essentially.

It has now been found that compounds of the general formula I below besides absorbing in the UV-B region show a good solubility in solvents commonly used in sunscreen agents.

Thus, an object of the present invention are compounds of the general formula I, 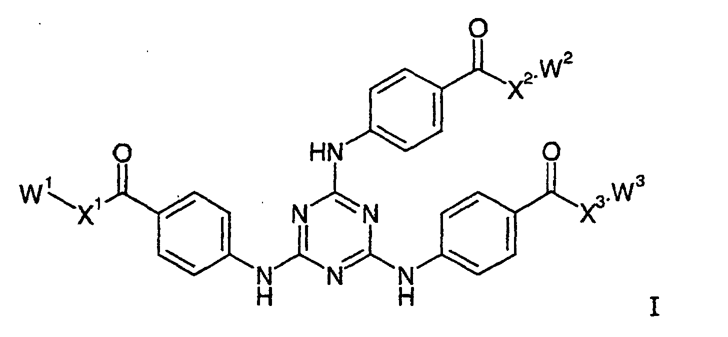 wherein
- W¹, W² and W³: each independently signifies C₁-C₂₀ alkyl or a group Sp-Sil;
- X¹, X² and X³: each independently signifies O or NH;
- Sp: signifies a spacer group;
- Sil: signifies a silane, an oligosiloxane or a polysiloxane moiety;
with the proviso that at least one of W¹,W² and W³ signifies SpSil.

Preferably W¹, W² and W³ signify SpSil; or W¹ and W² signify C₁-C₂₀ alkyl, preferably 2-ethylhexyl, and W³ signifies SpSil.

Preferably X¹, X² and X³ signify oxygen, or X¹ and X² signify oxygen and X³ signifies NH.

The term "spacer group" refers in this context to a C₃-C₁₂ divalent alkyl or alkylene chain which links the silane, oligosiloxane or polysiloxane moiety to the triazine residue. In said chains one or several carbon atoms may be replaced by oxygen atoms resulting in groups such as -C₁-C₆-alkyl-O-C₁-C₅-alkyl- e.g. -(CH₂)₂-O-(CH₂)₂- or -(CH₂)₄-O-(CH₂)₂-; -C₁-C₆-alkenyl-O-C₁-C₅-alkyl- e.g. -C(=CH₂)-(CH₂)₂-O-(CH₂)₄-; -C₁-C₄-alkyl-O-C₁-C₄-alkyl-O-C₁-C₂-alkyl -O-C₁-C₂-alkyl e.g -(CH₂)₂-O-(CH₂)₂-O-(CH₂)₂- and the like.

The term "C₃-C₁₂ divalent alkyl chain" embraces straight chain or branched saturated hydrocarbon residues such as 3-propylene, 2-propylene, 2-methyl-3-propylene, 3-butylene, 4-butylene, 4-pentylene, 5-pentylene, 6-hexylene, 12-dodecylene and the like.

The term "C₃-C₁₂ divalent alkylene chain" embraces straight chain or branched unsaturated hydrocarbon residues containing one or multiple double bonds such as 2-propen-2-ylene, 2-propen-3-ylene, 2-methyl-3-propenylene, 3-buten-3-ylene, 4-buten-3-ylene, 4-penten-4-ylene, 5-penten-4-ylene, (3-methyl)-penta-2,4-dien-4 or 5-ylene, 11-dodecen-11-ylene and the like.

Preferred spacer groups are: -(CH₂)₃-, -(CH₂)₄-, -(CH₂)₅-, -(CH)(CH₃)-(CH₂)-, -(CH₂)₂-CH=CH-, -C(=CH₂)-CH₂-, -C(=CH₂)-(CH₂)₂-O-(CH₂)₄-, (CH₂)₄-O-(CH₂)₂-.

The term "silane" refers in this context to a group SiR¹R²R³ wherein R¹, R² and R³ each independently signify C₁-C₆ alkyl, C₁-C₆ alkoxy or phenyl.

The term "alkyl" and "alkoxy" residues can be straight-chain or branched residues with the number of carbon atoms indicated such as e.g. methyl, ethyl, propyl, isopropyl, n-butyl, tert.butyl, 2-ethylhexyl, thexyl, (1,1,2- trimethylpropyl) and, respectively, methoxy, ethoxy, propoxy, isopropoxy, n-butoxy, tert.butoxy, 2-ethylhexoxy, thexoxy.

Examples for the group SiR¹R²R³ are: Si(CH₂-CH₃)₃, Si(CH₂-CH₂-CH₃)₃, Si(isopropyl)₃, Si(tert.butyl)₃, SiMe₂tert.butyl, SiMe₂thexyl, Si(OMe)₃, -Si(OEt)₃, SiPh₃ and the like, preferably Si(CH₂-CH₂-)₃- and Si(CH₂-CH₂-CH₂)₃.

The term "oligosiloxane" refers in this context to groups of the general formula SiR¹⁰ₘ(OSiR¹⁰₃)ₙ with m = 0, 1 or 2; n = 3, or 1 and m+n = 3; or a group of the general formula IIa, IIa' or IIb 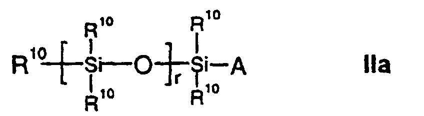 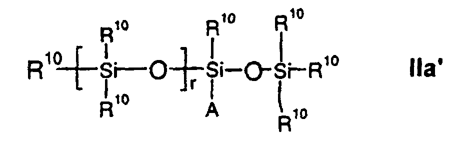 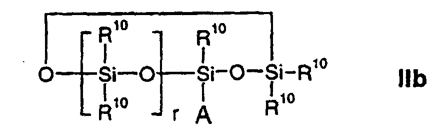 wherein
- A: signifies a bond to the spacer;
- R¹⁰: signifies C₁-C₆ alkyl or phenyl;
- r: signifies 1 to 9, preferably 1 to 3.

The term "polysiloxane" refers in this context to groups of the general formula IIIa or IIIb, 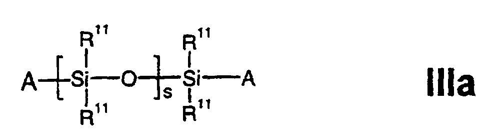 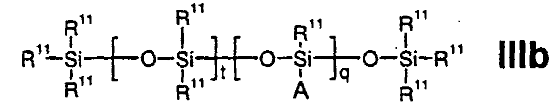 wherein
- A: is a bond to the spacer;
- R¹¹: signifies C₁-C₆ alkyl or phenyl;
- s: has a value of from 4 to 250, preferably 5 to 150;
- t: has a value of from 5 to 250, preferably 5 to 150, more preferably a statistical mean of about 60;
- q: has a value of from 1 to 30, preferably 2 to 10, more preferably a statistical mean of about 4.

The residues R¹⁰ and R¹¹ are preferably C₁-C₆ alkyl, more preferably C₁-C₄ alkyl, most preferably methyl.

In one particular embodiment of the invention W¹, W² and W³ signify Sp'-Sil'. Said embodiment refers to compounds of the formula Ia: 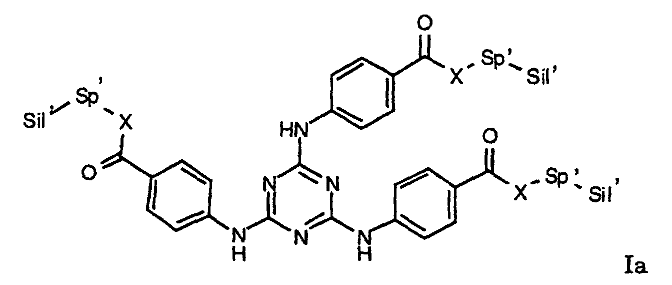 wherein
- X: signifies O or NH;
- Sp': signifies a straight-chain or branched saturated or single or multiple unsaturated hydrocarbon group of 3 to 12 carbon atoms;
- Sil': signifies the group SiR¹R²R³, wherein R¹, R² and R³ each independently signify C₁-C₆-alkyl, C₁-C₆-alkoxy or phenyl; or an oligosiloxane of the formula -SiMeₘ(OSiMe₃)ₙ, wherein Me is methyl and m is 0, 1 or 2, n is 1, 2 or 3 and m+n are 3; or an oligosiloxane of the formulae A, A' and B 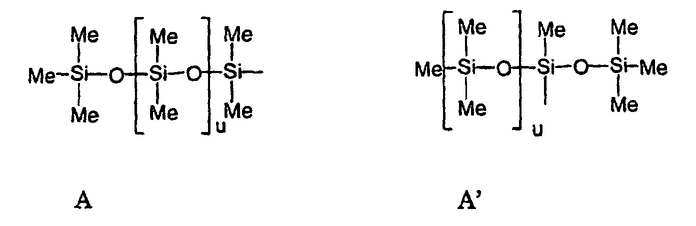 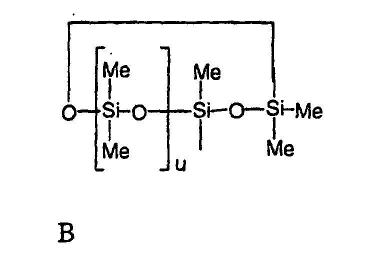
wherein Me is methyl and u is 0 to 6.

Especially preferred compounds of formula I are:
2,4,6-tri-anilino-p-{carbo-4'-(1,1,3,3,3-pentamethyldisiloxanyl)-1'-butyloxy)-1,3,5-triazine,
2,4,6-tri-anilino-p-(carbo-4'-(1,1,1,3,5,5,5-heptamethyl trisiloxanyl)-1'-butyloxy}-1,3,5-triazine,
2,4,6-tri-anilino-p-{carbo-2'-methyl-3'-(1,1,3,3,3-pentamethyl disiloxanyl)-1'-propyloxy)-1,3,5-triazine,
2,4,6-tri-anilino-p-{carbo-5'-(1,1,3,3,3-pentamethyl disiloxanyl)-1'-pentyloxy)-1,3,5-triazine,
2,4,6-tri-anilino-p-{carbo-3'-(1,1,3,3,3-pentamethyl disiloxanyl)-1'-propyloxy}-1,3,5-triazine,
2,4,6-tri-anilino-p-(carbo-4'-(triethly silyl)-1'-but-3-enyloxy}-1,3,5-triazine,
2,4,6-tri-anilino-p-(carbo-4'-(triethyl silyl)-1'-butyloxy}-1,3,5-triazine and
2,4,6-tri-anilino-p-{carbo-7'-(triethyl silyl)-4'-oxa-heptyloxy}-1,3,5-triazine,
2,4,6-tri-anilino-p-{carbo-7'-(1,1,3,3,3-pentamethyl disiloxanyl)-4'-oxa-heptyloxy}-1,3,5-triazine,
2,4-di-anilino-p-carbo-(2'-ethyl-hexyloxy)-6-anilino-p-{carbo-7'-(1,1,3,3,3-pentamethyl disiloxanyl)-4'-oxa-heptyloxy}-1,3,5-triazine,
2,4-di-anilino-p-carbo-(2'-ethyl-hexyloxy)-6-anilino-p-{carbo-7'-(1,1,1,3,5,5,5-heptamethyl trisiloxanyl)-4'-oxa-heptyloxy}-1,3,5-triazine,
2,4-di-anilino-p-carbo-(2'-ethyl-hexyloxy)-6-amino{N-(-2'-(1,1,1,3,5,5,5-heptamethyltrisiloxanyl)-allyl)-p-benzamidyl}-1,3,5-triazine, or a polysiloxane which corresponds in its statistical mean to the following formula:

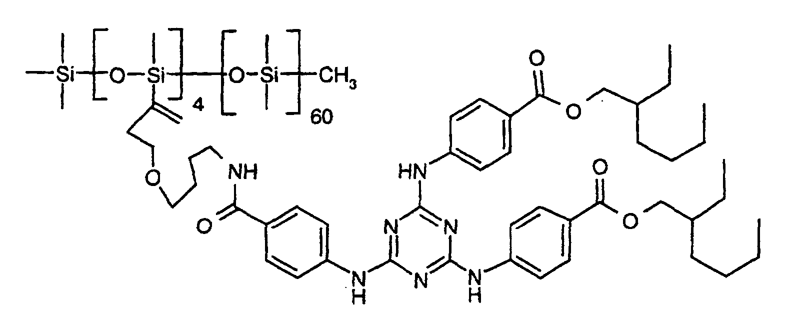

The compounds according to the present invention absorb UV radiations in the range from 290 to 320 nm and show an improved solubility in lipophilic solvents. Furthermore, they can easily be dispersed in emulsions and have relatively low tendency to crystallise in emulsions or on the skin. Thus, the compounds of the present invention can be used for the preparation of light screening compositions, especially for the preparation of a cosmetic composition useful for protecting human skin from sunlight radiation. These products can be applied in high concentrations which effect improved sun protection. Effects of aggregation and crystallisation which furnish a loss in efficacy are reduced in the emulsions and on the skin.

A further object of the present invention is a process for the preparation of the compounds of formula I.

Another object of the present invention is a light screening composition containing one or more compounds of formula I as sunscreen agents.

The synthesis of the compounds of formula I, wherein W¹, W² and W³ signify SpSil can be carried out according to the following steps I to III: 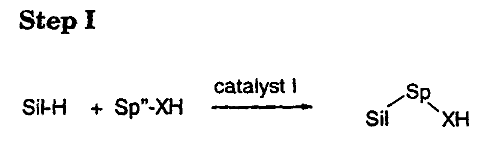 wherein Sil and X are as defined above; Sp" has the same meaning as Sp defined above, except that it has one degree of unsaturation more than Sp. In other words if Sp" has one double bond Sp is saturated and if Sp" has a triple bond or two double bonds Sp has one double bond. 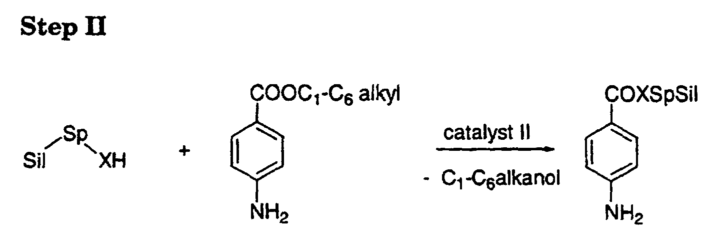 wherein Sil, Sp and X are as defined above. 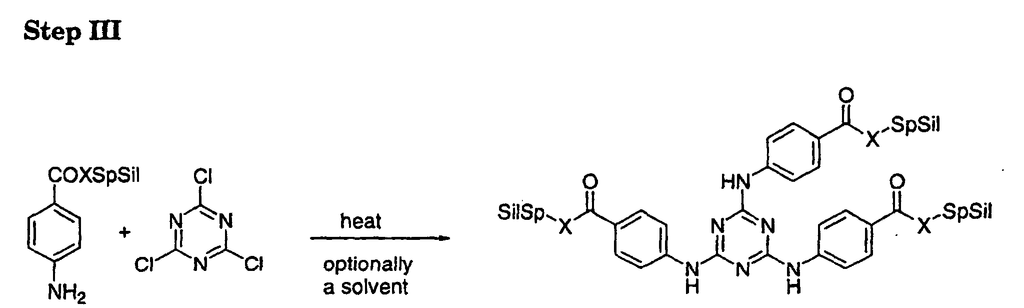 wherein Sil, Sp and X are as defined above.

The first step is a hydrosilylation reaction and can be performed according to methods known per se, such as at a temperature in the range of 0° to 200°C, preferably 40° to 110°C in the presence of a metal catalyst. The reaction is preferably carried out in an inert gas atmosphere, optionally in a solvent. Suitable catalysts are platin catalysts like metallic Pt on carbon, chloroplatinic acid, divinyl-tetramethyl-disiloxane platinum complex, or any other platinum complex; rhodium catalysts like metallic Rh 5% on carbon, bis(1,5-cyclooctadien)-di-Rh(I)-dichloride and the like; Mo, Ru, Pd, Cr, Fe, Co, Ni or Cu in their metallic form or as a complex. The catalysts can be homogeneous or heterogeneous. Most organic solvents can be used such as aromatic solvents, preferably toluene, xylene, pyridine; ether such as THF, dioxane and the like, aliphatic solvents such as dichloroethane, dichloromethane, chloroform, CCl₄, acetonitrile, DMF, DMSO, ethanol and the like.

The second step is a transesterification or an amide formation and can be performed according to methods known per se using a catalyst II. Thus, the reaction can be carried out at a temperature in the range of 50° to 250°C, optionally in a solvent. Organic solvents as listed under the first step can be used. Suitable catalysts are basic catalysts such as KOH, Na₂CO₃ and the like, acidic catalysts such as H₂SO₄, HCl and the like or a Lewis acid catalyst like e.g. tetraisopropyl orthotitanate.

The third step can be achieved just by treating slowly the product of the second step and cyanuric acid chloride at a temperature in the range of 20° to 280°C, preferably of 50° to 150°C without or with an appropriate solvent (e.g. toluene, xylene). Optionally a base can be present such as K₂CO₃, NaH and the like.

The sequence of steps can be freely interchanged, e.g. it is possible to start with step III by treating cyanuric acid chloride with C₁₋₆-alkyl 4-aminobenzoic acid ester followed by a transesterification or an amide formation (step II) and finishing with the hydrosilylation step I.

The synthesis of the compounds of formula I, wherein one or two of the groups W¹, W² and W³ signify C₁-C₂₀ alkyl and the remaining groups of W¹, W² and W³ signify SpSil can also be carried out comparable to the above reaction scheme. Cyanuric acid chloride is treated either at first with 4- aminobenzoic acid C₁-C₂₀ alkyl ester at a temperature in the range of 0° to 40°C, and then with a compound of the formula A 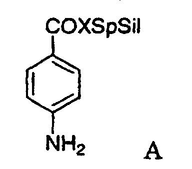 wherein X, Sp and Sil are as defined above at a temperature in the range of 20° to 280°C, or cyanuric acid chloride is treated at first with a compound of the formula A at a temperature in the range of 0° to 40°C and then with 4-aminobenzoic C₁-C₂₀ alkyl ester at a temperature in the range of 20° to 280°C.

Compounds of the formula I, wherein Sil is a polysiloxane are preferably prepared according to the following schema: 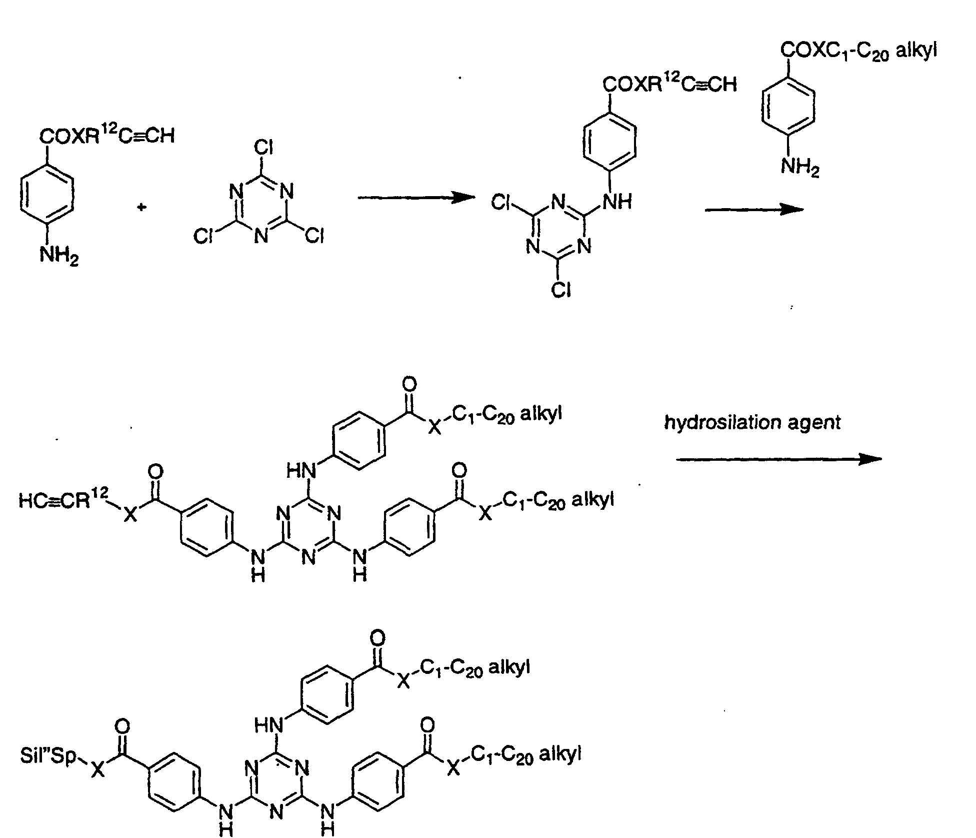 wherein X and Sp are as defined above, Sil" is a polysiloxane as defined above and R¹²is a C₁-C₁₀ divalent alkyl chain or a C₃-C₁₀ divalent alkyl chain optionally interrupted by at least one oxygen atom.

Compounds according to the invention are colourless or slightly yellowish liquids, semiliquids or crystalline compounds. They are especially qualified as light screening agents because of their high absorption of UV-B light, their good solubility in organic solvents, especially in solvents commonly used in cosmetic industry and because of their easy and cheap access. They can be combined with one or more known UV -B and/or UV-A filters.

The preparation of novel light screening agents, especially of preparations for skin protection and, respectively, sunscreen preparations for everyday cosmetics, comprises incorporating a compound of formula I in a cosmetic base which is usual for light screening agents. Where convenient, other conventional UV-A, and respectively, UV-B filters can also be combined during this incorporation. Said combinations of UV filters can show a synergistic effect. The preparation of said light screening agents is well known to the skilled artisan in this field. The amount of compounds of the general formula I and other known UV-filters is not critical. Suitable amounts are about 0.5 to about 12%.

Suitable UV B filters, i.e. substances having absorption maxima between about 290 and 320 nm, are for example the following organic compounds which belong to the widest classes of substance:
--- p-Aminobenzoic acid derivatives such as ethyl, propyl, butyl, isobutyl, octyldimethyl, amyldimethyl, ethoxylated ethyl, propoxylated ethylglyceryl or ethylglycosyl p-aminobenzoate and the like;
--- Acrylates such as 2-ethylhexyl 2-cyano-3,3-diphenylacrylate (octocrylene), ethyl 2-cyano-3,3-diphenylacrylate and the like;
--- Aniline derivatives such as methyl anilinum methosulfate and the like;
--- Anthranilic acid derivatives such as menthyl anthranilate and the like;
--- Benzophenone derivatives such as benzophenone-1 to benzophenone-12 and the like.
--- Camphor derivatives such as methyl benzylidene camphor, 3-benzylidene camphor, camphor benzalkonium methosulfate, polyacrylamidomethyl benzylidene camphor, sulfo benzylidene camphor, sulphomethylbenzylidene camphor, therephthalidene dicamphor sulfonic acid and the like;
--- Cinnamate derivatives such as octyl methoxycinnamate or ethoxyethyl methoxycinnamate, diethanolamine methoxycinnamate, isoamyl methoxycinnamate and the like as well as cinnamic acid derivatives bond to siloxanes;
--- Gallic acid such as digalloyl trioleate and the like;
--- Imidazole derivatives such as phenyl benzimidazole sulfonic acid and the like;
--- Salicylate derivatives such as isopropylbenzyl, benzyl, butyl, octyl, isooctyl or homomenthyl salicylate and the like;
--- Triazole derivatives such as drometriazole, hydroxydibutylphenyl-, hydroxydiamylphenyl-,hydroxyoctylphenyl- or hydroxyphenylbenztriazole and the like;
--- Triazone derivatives such as octyl triazone and the like; and
--- Pigments such as microparticulated TiO₂, ZnO and the like.

The formulation may further contain UV-A filters such as
--- Dibenzoylmethane derivatives such as 4-tert. butyl-4'-methoxydibenzoyl-methane and the like;
--- Triazine compounds as described in the European Patent Publications EP 0693483 A1, EP 0704437 A2, EP 0704444 A1 and EP 0780382 A1;
--- Organosiloxane compounds as described in the European Patent Publications EP 0538431 B1, EP 0709080 A1 and EP 0358584B1;
--- Malonates such as described in the European Patent Application 98114262.3

Especially useful for effective UV absorption are combinations with metallo-oxy nano pigments like e.g. titanoxide, zincoxide, ceroxide, zircon oxide, ferrous oxide and mixtures thereof, the diameter of which is < 100 nm.

The following Examples 1-15 further illustrate the invention but do not limit its scope in any manner.

Examples 8 and 9 are comparative examples. Example 16 refers to a light screening composition.

### Example 1:

### Preparation of 2,4,6-tri-anilino-p-{carbo-4'-(1,1,3,3,3-pentamethyldisiloxanyl)-1'-butyloxy}-1,3,5-triazine.

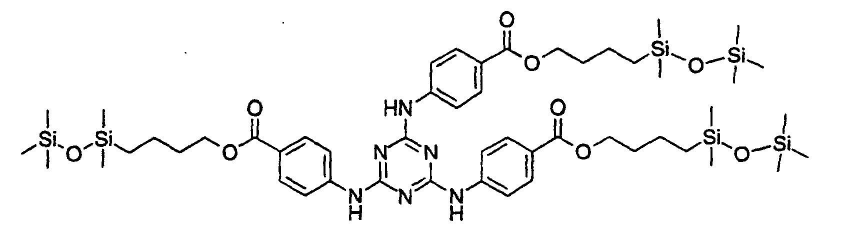

a) First step: preparation of 4-(1,1,3,3,3-pentamethyl disiloxanyl)-1-butanol.
   A 50 ml reaction flask was charged with 10.3 ml 3-butene-1-ol and a catalytic amount of divinyl-tetramethyl disiloxane platinum complex under inert atmosphere and heated to 60°C. 19.5 ml of pentamethyl disiloxane was slowly added through a dropping funnel. The reaction mixture was stirred at 75 to 80°C for three hours, followed by distillation at 110 to 115°C/38 x 10² Pa over a 10 cm column. Yield 18.9 g (86% of the theory) of a clear liquid.
b) Second step: preparation of 4-aminobenzoic acid 4-(1,1,3,3,3-pentamethyl disiloxanyl) butyl ester.
   A 50 ml reaction flask was charged with 9.9 g of ethyl 4-aminobenzoic acid ester, 15.3 g of 4-(1,1,3,3,3-pentamethyl disiloxanyl)-1-butanol and 0.06 ml of tetra- isopropyl orthotitanate and heated to 110°C/90 x 10² Pa for 7 h.
   The ethanol formed was subsequently distilled off. The product was fractionated at 205°C/0.06 x 10² Pa to yield 12,6 g (62% of the theory) of a clear liquid.
c) Third step: preparation of 2,4,6-tri-anilino-p-{carbo-4'-(1,1,3,3,3-pentamethyl disiloxanyl)-1'-butyloxy}-1,3,5-triazine.
   In a 400 ml reaction flask 10.2 g of 4-aminobenzoic acid 4-(1,1,3,3,3-pentamethyl disiloxanyl) butyl ester were dissolved in 150 ml of toluene and cooled to 0°C. A solution of 1.83 g of cyanuric acid chloride in 60 ml of toluene was slowly added within 20 min. Then the reaction mixture was gently heated to reflux temperature and stirred at this temperature for 48 hours. The solvent was then removed using a rotation evaporator and the residue was chromatographed over SiO₂ in hexane/EtOAc = 9:1. 3.5 g of a semicrystalline product was isolated. UV 308 nm (133230), m.p. 86 - 87°C.
d) Measurement of solubility in Cétiol LC (cocoyl caprylate caprate) and Crodamol DA® (diisopropyl adipate) .
   Oversaturated solutions of 2,4,6-tri-anilino-p-{carbo-4'-(1,1,3,3,3-pentamethyl disiloxanyl)-1'-butyloxy}-1,3,5-triazine in the above solvents were prepared and treated in an ultrasound bath for five minutes. After standing over night at 25°C the solution was filtered through a microfilter (Millipore, pore size 0.5 µm), followed by UV measurement in CH₂Cl₂. The extinctions were compared with the extinction of the pure compound. The solubilities were found to be 11.2% in Cétiol LC® and >26% in Crodamol DA®.
   Examples 2 to 5 were prepared following the procedure as described in Example 1.

### Example 2:

### Preparation of 2,4,6-tri-anilino-p-{carbo-4'-(1,1,1,3,5,5,5-heptamethyl trisiloxanyl)-1'-butyloxy}-1,3,5-triazine.

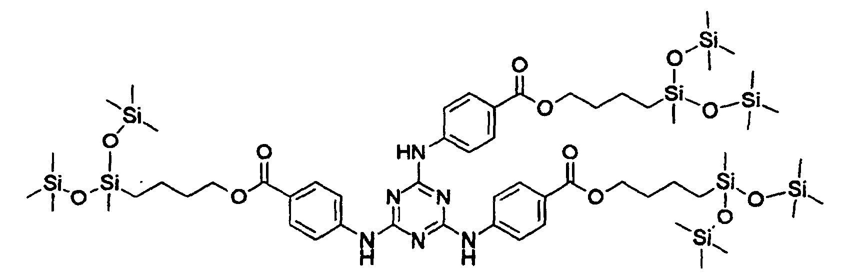

a) First step: preparation of 4-(1,1,1,3,5,5,5-heptamethyl trisiloxanyl)-1-butanol using 1,1,1,3,5,5,5-heptamethyl trisiloxane. After destillation at 74 - 78°C/0.1 x 10² Pa 83% of a liquid product was obtained
b) Second step: preparation of 4-aminobenzoic acid 4-(1,1,1,3,5,5,5-heptamethyl trisiloxanyl) butyl ester by reacting 4-(1,1,1,3,5,5,5-heptamethyl trisiloxanyl)-1-butanol with ethyl 4-aminobenzoic acid ester. After chromatography over SiO₂ in hexane/EtOAc = 1.1 and evaporation of the starting material, a dear yellow oil was obtained in 43% yield.
c) Third step: preparation of 2,4,6-tri-anilino-p-{carbo-4'-(1,1,1,3,5,5,5-heptamethyl trisiloxanyl)-1'-butyloxy}-1,3,5-triazine by heating to reflux 4-aminobenzoic acid 4-(1,1,1,3,5,5,5-heptamethyl trisiloxanyl) butyl ester and cyanuric acid chloride. After chromatography 37% of the product was obtained. UV 308 nm (120459), m.p. 115 - 118°C.
d) Measurement of solubility in Cétiol LC® and Crodamol DA®: 19.1% in Cétiol LC® and 35.5% in Crodamol DA®.

### Example 3:

### Preparation of 2,4,6-tri-anilino-p-{carbo-2'-methyl-3'-(1,1,3,3,3-pentamethyl disiloxanyl)-1'-propyloxy}-1,3,5-triazine.

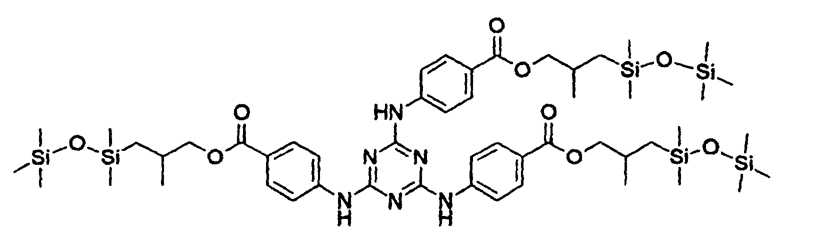

a) First step: preparation of 3-(1,1,3,3,3-pentamethyl disiloxanyl)-2-methyl-1-propanol using 2-methallyl alcohol instead of 3-butene-1-ol. After destination at 105°C/40 x 10² Pa 81% of a liquid product was obtained.
b) Second step: preparation of 4-aminobenzoic acid 2-methyl-3-(1,1,3,3,3-pentamethyl disiloxanyl) propyl ester by reacting 3-(1,1,3,3,3-pentamethyl disiloxanyl)-2-methyl-1-propanol. A clear yellow oil was obtained in 27% yield.
c) Third step: preparation of 2,4,6-tri-anilino-p-{carbo-2'-methyl-3'-(1,1,3,3,3-pentamethyl disiloxanyl)-1'-propyloxy}-1,3,5-triazine by heating to reflux 4-aminobenzoic acid 2-methyl-3-(1,1,3,3,3-pentamethyl disiloxanyl) propyl ester and cyanuric acid chloride. After chromatography 33% of the product was obtained. UV 308 nm (109184), m.p. 118 - 120°C.
d) Measurement of solubility in Cétiol LC® and Crodamol DA®: 16.8% in Cétiol LC® and >34% in Crodamol DA®.

### Example 4:

### Preparation of 2.4.6 -tri -anilino-p-{carbo-5'-(1,1,3,3,3-pentamethyl disiloxanyl)-1'-pentyloxy}-1,3,5-triazine.

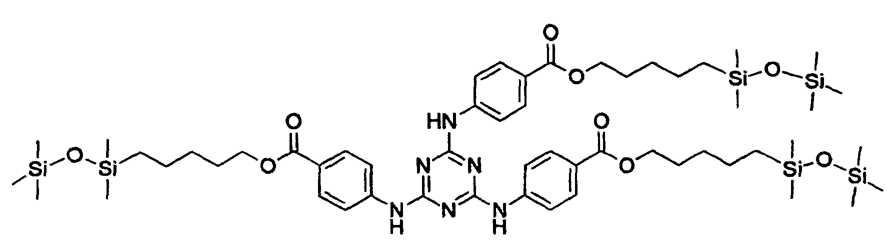

a) First step: preparation of 5-(1,1,3,3,3-pentamethyl disiloxanyl)-1-pentanol by using 4-penten-1-ol instead of 3-butene-1-ol. After destillation at 124 - 125°C/39 x 10² Pa 88% of a liquid product was obtained.
b) Second step: preparation of 4-aminobenzoic acid 5-(1,1,3,3,3-pentamethyl disiloxanyl) pentyl ester by reacting 5-(1,1,3,3,3-pentamethyl disiloxanyl)-1-pentanol instead of 4-(1,1,3,3,3-pentamethyl disiloxanyl)-1-butanol. A clear yellow oil was obtained in 63% yield.
c) Third step: preparation of 2,4,6-tri-anilino-p-{carbo-5'-(1,1,3,3,3-pentamethyl disiloxanyl)-1'-pentyloxy}-1,3,5-triazine by heating to reflux 4-aminobenzoic acid 5-(1,1,3,3,3-pentamethyl disiloxanyl) pentyl ester and cyanuric acid chloride. After chromatography 20% of the product was obtained. UV 308 nm (137085), m.p. 119.5 - 121.5°C.
d) Measurement of solubility in Cétiol LC® and Crodamol DA®: 6.2% in Cétiol LC® and 13% in Crodamol DA®.

### Example 5:

### Preparation of 2,4,6-tri-anilino-p-{carbo-3'-(1,1,3,3,3-pentamethyl disiloxanyl)-1'-propyloxy}-1,3,5-triazine.

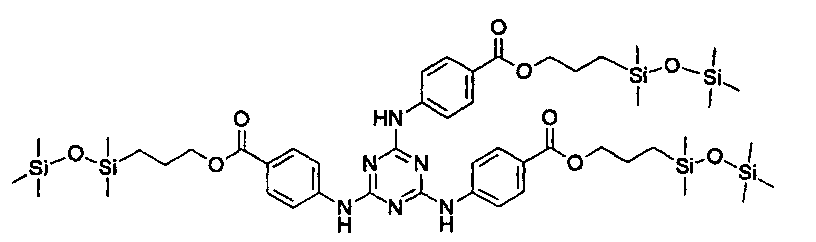

a) First step: preparation of 3-(1,1,3,3,3-pentamethyl disiloxanyl)-1-propanol using allyl alcohol instead of 3-butene-1-ol. After destillation at 99 - 101°C/41 x 10² Pa 85% of a liquid product was obtained.
b) Second step: preparation of 3-aminobenzoic acid 3-(1,1,3,3,3-pentamethyl disiloxanyl) propyl ester by reacting 3-(1,1,3,3,3-pentamethyl disiloxanyl)-1-propanol. A dear yellow oil was obtained in 14% yield.
c) Third step: preparation of 2,4,6-tri-anilino-p-{carbo-3'-(1,1,3,3,3-pentamethyl disiloxanyl)-1'-propyloxy}-1,3,5-triazine by heating to reflux 3-aminobenzoic acid 3-(1,1,3,3,3-pentamethyl disiloxanyl) propyl ester and cyanuric acid chloride. After chromatography 32% of the product was obtained. UV 308 nm (111664), m.p. 125 - 127°C.
d) Measurement of solubility in Cétiol LC® and Crodamol DA®: 4.3% in Cétiol LC® and >16% in Crodamol DA®.

### Example 6

### Preparation of 2,4,6-tri-anilino-p-{carbo-4'-(triethly silyl)-1'-but-3-enyloxy}-1,3,5-triazine

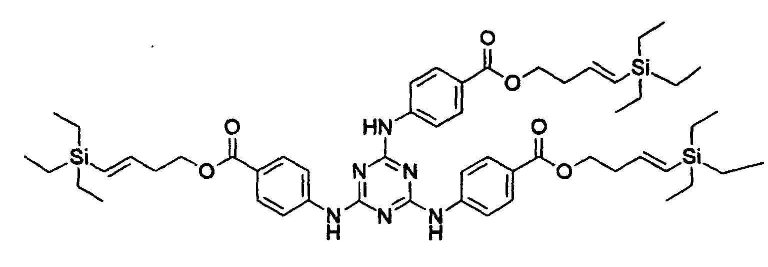

a) First step: preparation of 4-(triethylsilyl)-1-but-3-enol
   A 50 ml reaction flask was charged with 1 butene-3-ol and a catalytic amount of bis(1,5-cyclooctadien)-di-Rh(I)-dichloride and triphenylphosphine under inert atmosphere. Triethylsilane was slowly added through a dropping funnel. The reaction mixture was stirred at room temperature for 72 h. 86% of a yellow liquid was obtained.
b) Second step: preparation of 4-aminobenzoic acid-(triethylsilyl) 1-but-3-enylester by reacting 4-aminobenzoic acid ester and 4-(triethylsilyl)-1-but-3-enol. Yield: 69% of a clear liquid.
c) Third step: preparation of 2,4,6-tri-anilino-p-{carbo-4'-(triethly silyl)-1'-but-3-enyloxy}-1,3,5-triazine.

### Example 7

Example 7 was prepared analog to Example 6.

### Preparation of 2,4,6-tri-anilino-p-{carbo-4'-(triethyl silyl)-1'-butyloxy}-1,3,5-triazine

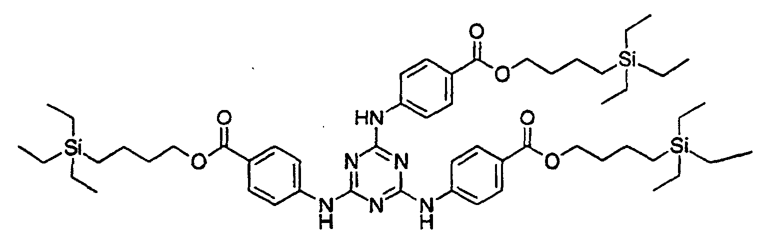

a) First step: preparation of 4-(triethylsilyl)-lbutanol.
b) Second step: preparation of 4-aminobenzoic acid-(triethylsilyl) butylester.
c) Third step: preparation of 2,4,6-tri-anilino-p-(carbo-4'-(triethyl silyl)-1'-butyloxy}-1,3,5-triazine.

### Example 8

### Preparation of 2,4,6-tri-anilino-(p-carboethoxy)-1,3,5-triazine as a reference compound without any silyl groups.

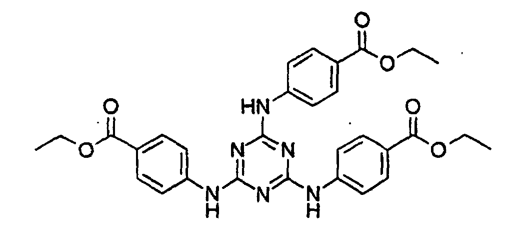

A 750 ml reaction flask was charged with 13.9 g of ethyl-p-amino-benzoate dissolved in 300 ml of p-cymene. A solution of 4.85 g of cyanuric acid chloride in 150 ml of p-cymene was slowly added within 20 min. A white suspension was formed. Then the reaction mixture was gently heated to reflux temperature (170°C) and stirred at this temperature for 20 h. The reaction mixture was cooled to 0°C, filtered and the residue was washed with MTBE and recrystalised in toluene to yield 12.3g (82%) of white crystals. UV 308 nm (133'374), m.p. 218 - 220°C, which predicted a low solubility.

### Measurement of solubility in Cétiol LC® and Crodamol DA®:

The solubilities were determined as described in Example 1 and were found to be 0.02% in Cétiol LC® and 0.2% in Crodamol DA®.

### Example 9

### Measurement of solubility of UVINUL T-150 in Cétiol LC® and Crodamol DA® for comparison:

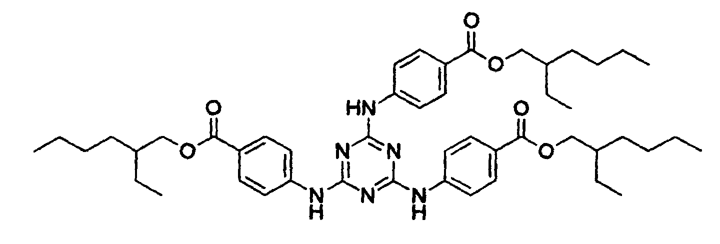

The solubilities were determined for the commercial product Uvinul T-150, as a reference, as described in Example 1. UVINUL T-150 was the best soluble compound within the known series The solubilities were found to be 3.7 - 4.2% in Cétiol LC® and 10% in Crodamol DA®, which was lower then for the examples according to the invention. The m.p. was 126 - 127.5°C.

### Example 10

### Preparation of 2,4,6-tri-anilino-p-{carbo-7'-(triethyl silyl)-4'-oxa-heptyloxy}-1,3,5-triazine.

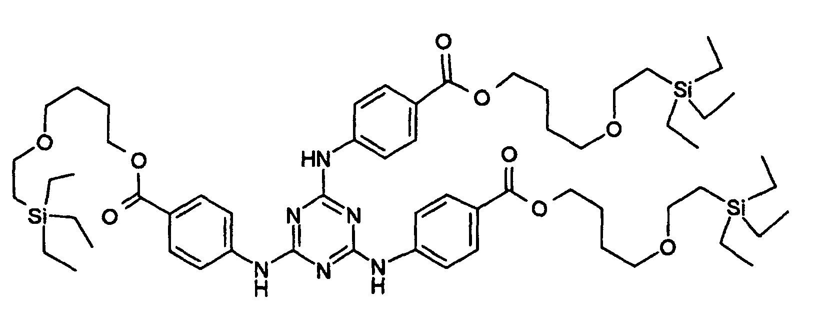

a) 4-(2-Triethylsilanyl-ethoxy)-butanol:
   A 50 ml reaction flask was charged with 11.6 ml (100 mmol) of 1,4-butanediol-mono vinylether and a catalytic amount of divinyl-tetramethyl disiloxane platinum complex under inert atmosphere and heated to 60°C. 10.4 g (90 mmol) of triethylsilane was slowly added through a dropping funnel. The exothermic reaction mixture was stirred at 75°C for 18 hours, followed by distillation at 105 to 107°C / 20 m⁻¹ kg s⁻² over a 10 cm Vigreux column.
   Yield 15.2 g (66% of the theory) of a clear liquid.
   Purity 98.7% according gas chromatography.
b) 4-Aminobenzoic acid 4-(2-triethylsilanyl-ethoxy)-butyl ester:
   The same reaction was performed as in example 1b, using the product of the above reaction instead of 4-(1,1,3,3,3-pentamethyl disiloxanyl)-l-butanoL A clear yellow oil was received in 65% yield after chromatography.
c) 2,4,6-Tri-anilino-p-{carbo-7'-(triethyl silyl)-4'-oxa-heptyloxy}-1,3,5-triazine.
   The reaction procedure of example 1c was repeated using the p-amino benzoic acid ester received above instead of p-aminobenzoic acid 4-(pentamethyl disiloxanyl) butyl ester. After chromatography 79% of the product is obtained. UV 308 nm (ε=117'723), m.p. 79 - 81°C.
d) Measurement of solubility in cosmetic solvents:
   The solubilities were determined as described in example 1 and were found to be 31% in Cétiol LC® and 45% in Crodamol DA®.

### Example 11

### Preparation of 2,4,6-tri -anilino-p-{carbo-7'-(1,1,3,3,3-pentamethyl disiloxanyl)-4'-oxa-heptyloxy}-1,3,5-triazine.

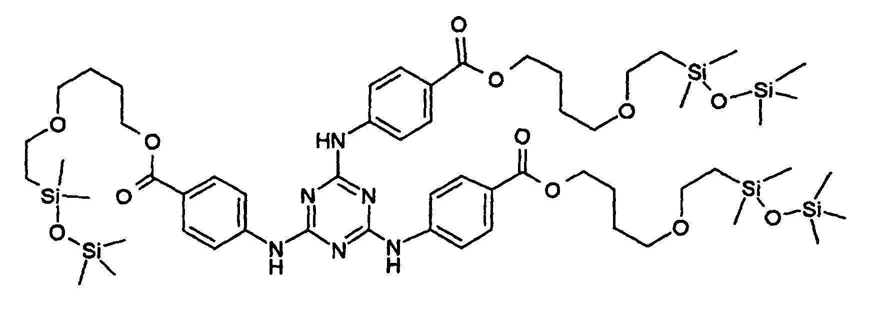

a) 7-(1,1,3,3,3-Pentamethy-disiloxanyl)-4-oxa-heptan-1-ol:
   A 50 ml reaction flask was charged with 16 ml of 1,4-butanediol-vinylether and a catalytic amount of divinyl-tetramethyl disiloxane platinum complex under inert atmosphere and heated to 60°C. 22.8 ml of pentamethyl disiloxane was slowly added through a dropping funnel. The reaction mixture was stirred at 75 to 80°C for 18 hours, followed by distillation at 85 to 87°C / 20 m⁻¹ kg s⁻² over a 10 cm column. Yield 29 g (85% of the theory) of a dear liquid.
b) 4-Nitrobenzoic acid 7-(1,1,3,3,3-pentamethy-disiloxanyl)-4-oxa-heptyl ester
   A 100 ml reaction flask was charged with 20 g of the above silylated alcohol in 34 ml of pyridine and vigorously stirred. 22.5 g of p-nitrobenzoic acid chloride was slowly added within 20 min. The reaction mixture was heated to 60°C. and stirred for one hour. Then it was pored in ice water and extracted with CH₂Cl₂. The combined organic phases were washed with In HCl and saturated NaHCO₃ solution, dried over Na₂SO₄ and concentrated to yield 20.2 g (65%) of a yellow liquid. MS: 370, 298, 150, 147, 120(100%).
c) 4-Aminobenzoic acid 7-(1,1,3,3,3-pentamethy-disiloxanyl)-4-oxa-heptyl ester:
   A 600 ml hydrogenation autoclave was charged with 20 g of the above ester in 280 ml of methanol and 0.7 g of acetic acid and 3 g of Raney Ni catalyst. This mixture was hydrogenated for 18 hours an room temperature and a pressure of 6.895.10⁵ m⁻¹. kg. s⁻² . Then the mixture was filtered, distributed between ethyl acetate and water and the organic phase was concentrated to yield 93% of a yellow liquid. MS: 383(M⁺), 340, 268, 208, 147, 120(100%).
d) 2,4,6-Tri-anilino-p-{carbo-7'-(1,1,3,3,3-pentamethyl disiloxanyl)-4'-oxa-heptyloxy}-1,3,5-triazine.
   The reaction procedure of example 1c was repeated using p-amino benzoic acid ester received above instead of p-aminobenzoic acid 4-(pentamethyl disiloxanyl) butyl ester. After chromatography 60% of the product is obtained UV 308 nm (ε=124'248), m.p. 74 - 76°C.
e) Measurement of solubility in cosmetic solvents:
   The solubilities were determined as described in example 1 and were found to be 28% in Cétiol LC® and 29.4% in Crodamol DA®.

### Example 12

### Preparation of 2,4-di-anilino-p-carbo-(2'-ethyl-hexyloxy)-6-anilino-p-{carbo-7'-(1,1,3,3,3-pentamethyl disiloxanyl)-4'-oxa-heptyloxy}-1,3,5-triazine.

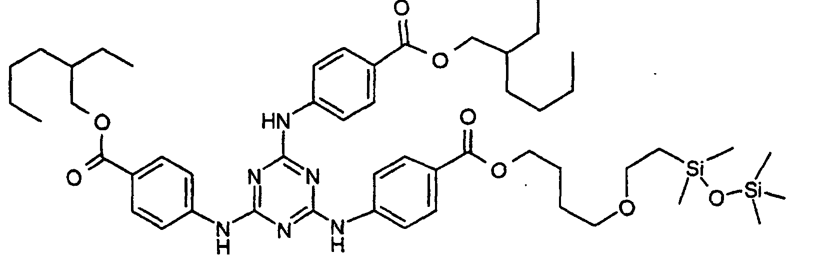

a) 2,4-Di-anilino-p-carbo-(2'-ethyl-hexyloxy)-6-chloro-1,3,5-triazine.
   A 100 ml reaction flask was charged with 0.92 g of cyanuric acid chloride in 50 ml of THF. A solution of 1.2 g of 2-ethyl-hexyl p-aminobenzoate and 0.85 ml of diisopropyl ethylamine in 25 ml THE was slowly added at 0°C. The reaction was slowly heated to 45°C and again a solution of 1.2 g of 2-ethylhexyl p-aminobenzoate and 0.85 ml of diisopropyl ethylamine in 25 ml THF was slowly added. After 18 hours the reaction mixture was distributed between water and ethyl acetate. The organic phases were dried and concentrated to yield 2.6 g of a crystalline product which was chromatographed on silica gel in hexane : ethyl acetate = 7:3. 1.8 g (56%) of a white crystalline material was isolated. MS: 609 (M⁺), 497, 385 (100%).
b) 2,4-Di-anilino-p-carbo-(2'-ethyl-hexyloxy)-6-anilino-p-{carbo-7'-(1,1,3,3,3-pentamethyl disiloxanyl)-4'-oxa-heptyloxy}-1,3,5-triazine.
   A 25 ml reaction flask was charged with 0.61 g of the above di-anilinotriazine in 15 ml of toluene and heated to 65°C. A solution of 0.38 g of 4-aminobenzoic acid 7-(1,1,3,3,3-pentamethy-disiloxanyl)-4-oxa-heptanyl ester (see example lic) in 3 ml of toluene was added and the reaction was stirred for 7 hours at 75°C. Then the reaction mixture was concentrated and chromatographed on silica gel in hexane : ethyl acetate = 7:3 to yield 46% of a white crystalline product. UV 308 nm (ε=112'526), m.p. 107 - 109°C.
c) Measurement of solubility in cosmetic solvents:
   The solubilities were determined as described in example 1 and were found to be 22% in Cétiol LC® and >36% in Crodamol DA®.

### Example 13

### Preparation of 2,4-di-anilino-p-carbo-(2'-ethyl-hexyloxy)-6-anilino-p-{carbo-7'-(1,1,1,3,5,5,5-heptamethyl trisiloxanyl)-4'-oxa-heptyloxy}-1,3,5-triazine.

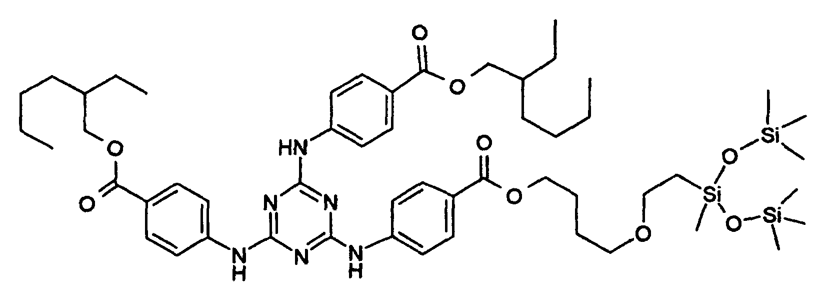

a) 7-(1,1,1,3,5,5,5-Heptamethyl trisiloxanyl)-4-oxa-heptanol:
   A 50 ml reaction flask was charged with 11.6 g of 1,4-butanediol-vinylether and a catalytic amount of divinyl-tetramethyl disiloxane platinum complex under inert atmosphere and heated to 80°C. 20 g of heptamethyl trisiloxane was slowly added through a dropping funnel. The reaction mixture was stirred at 85°C for 4 hours, followed by distillation at 110 to 112°C / 25 m⁻¹ kg s⁻² over a 10 cm column. Yield 22.1 g (73% of the theory) of a clear liquid.
b) 4-Nitrobenzoic acid 7-(1,1,1,3,5,5,5-heptamethyl trisiloxanyl)-4-oxa-heptyl ester:
   A 25 ml reaction flask was charged with 8 g of the above silylated alcohol in 10.5 ml of pyridine and vigorously stirred. 7 g of p-nitrobenzoic acid chloride was slowly added within 20 min. The reaction mixture was heated to 60°C. and stirred for one hour. Then it was pored into ice water and extracted three times with CH₂Cl₂. The combined organic phases were washed with 1-n HCl and saturated NaHCO₃ solution, dried over Na₂SO₄ and concentrated to yield 9.9 g (86%) of a yellow liquid.
c) 4-Aminobenzoic acid 7-(1,1,1,3,5,5,5-heptamethyl trisiloxanyl)-4-oxa-heptyl ester:
   A 300 ml hydrogenation autoclave was charged with 9.7 g of the above ester in 115 ml of methanol and 0.3 g of acetic acid and 1.2 g of Raney Ni catalyst. This mixture was hydrogenated for 18 hours at room temperature and a pressure of 6.895 x 10⁵ m⁻¹ kg s⁻².
   Then the mixture was filtered, distributed between ethyl acetate and water and the organic phase was concentrated to yield 8.3 g (91%) of a yellow liquid. MS: 457(M⁺), 342, 268, 221, 208, 137, 120(100%).
d) 2,4-Di-anilino-p-carbo-(2'-ethyl-hexyloxy)-6-anilino-p-{carbo-7'-(1,1,1,3,5,5,5-heptamethyl trisiloxanyl)-4'-oxa-heptyloxy) -1,3,5-triazine.
   A 25 ml reaction flask was charged with 0.85 g of 2,4-di-anilino-p-carbo-(2'-ethyl-hexyloxy)-6-chloro-1,3,5-triazine (preparation was described in example 12a) dissolved in 15 ml of toluene and heated to 65°C. A solution of 0.7 g of 4-aminobenzoic acid 7-(1,1,1,3,5,5,5-heptamethyl trisiloxanyl)-4-oxa-heptanyl ester (see above) in 3 ml of toluene was added and the reaction was stirred for 7.5 hours an 75°C. Then the reaction mixture was concentrated and chromatographed on silica gel in hexane : ethyl acetate = 7:3 to yield 1.13 g (79%) of a white crystalline product. UV 308 nm (ε=105'587), m.p. 103 - 105°C.
e) Measurement of solubility in cosmetic solvents:
   The solubilities were determined as described in example 1 and were found to be 27% in Cétiol LC® and >47% in Crodamol DA®.

### Example 14

### Preparation of 2,4-di-anilino-p-carbo-ethyl-hexyloxy)-6-amino{N-(-2'-(1,1,1,3,5,5,5-heptamethyl trisiloxanyl)-allyl)-p-benzamidyl}-1,3,5-triazine.

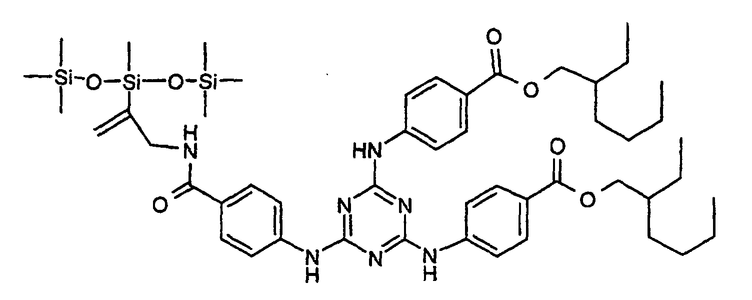

a) 4-Nitrobenzoic acid propargylamide:
   A 500 ml reaction flask is charged with 19.8 ml of propargylamine and 62 ml of triethylamine in 150 ml of methyl-tert.butyl ether (MTBE). 55.2 g of p-nitrobenzoic acid chloride is dissolved in 100 ml of MTBE and slowly added within 20 min. The reaction mixture is vigorously stirred for 90 min. and then heated to 60°C. for further 30 min. Then it is filtered and the residue washed with water, again filtered and recrystallised in acetonitrile to yield 40.7 g of yellow crystals.
b) 4-Aminobenzoic acid propargylamide:
   A 1 1 reaction flask was charged with 33.5 g of 4-nitrobenzoic acid propargylamide dissolved in 410 ml of methanol and 410 ml of concentrated aq. hydrochloric acid. 81.8 g of tin powder was added and the reaction was heated to 40°C for 165 min. Then the reaction mixture was poured in a solution of 410 g of NaOH in 1640 ml of ice water and the methanol was distilled off and filtered when hot to remove the inorganic material. The product crystallised from this solution. It was recrystallised from ethanol /water to yield 21.5 g (75%) of the desired material. m.p. 122-125°C.
c) 4-(4,6-Dichloro-(1,3,5)triazin-2-ylamino)-N-(propargyl-p-benzamide.
   A 75 ml reaction flask was charged with a cooled solution of 1.85 g of cyanuric acid chloride and 0.88 g of NaHCO₃ in 20 ml of acetone. A solution of 1.77 g of 4-aminobenzoic acid propargylamide in 6 ml of acetone was slowly added at 0°C. The yellowish suspension was stirred for 30 min. Then 9 ml of water was added and the product was filtered off to yield 2.5 g of a yellowish powder. MS: 322(M⁺), 321, 292, 267(100%).
d) 2,4-Di-anilino-p-carbo-(2'-ethyl-hexyloxy)-6-amino-{N-(propargyl)-p-benzamidyl}-1,3,5-triazine.
   A 25 ml reaction flask was charged with 1.04 g of 2-ethyl-hexyl p-aminobenzoate and 0.64 g of 4-(4,6-dichloro-(1,3,5)triazin-2-ylamino)-N-(propargyl-p-benzamide received above dispersed in 12 ml of Xylene and refluxed for six hours. The reaction mixture was cooled to 0°C. and the product was filtered off and once more recrystallised in toluene. 0.98 g of a slightly yellow powder was obtained. UV 306 nm (111'436), MS: 747(100%, M⁺).
e) 2,4-Di-anilino-p-carbo-(2'-ethyl-hexyloxy)-6-amino{N-(-2'-(1,1,1,3,5,5,5-heptamethyl trisiloxanyl)-allyl)-p-benzamidyl}-1,3,5-triazine.
   A 25 ml reaction flask was charged with 1.23 g of the triazine received above in 19 ml of toluene, 0.45 g of 1,1,1,3,5,5,5-heptamethyl trisiloxane and a catalytic amount of divinyl-tetramethyl disiloxane platinum complex under inert atmosphere. The reaction mixture was heated to 95°C. for five days, washed with water, concentrated and chromatographed in hexane ethyl acetate =7:3. 0.25 g of off white crystals were isolated. UV 306 nm (ε=106'517), m.p. 76-80°C.
f) Measurement of solubility in cosmetic solvents:
   The solubilities were determined was described in example 1 and were found to be >24% in Cétiol LC® and >37% in Crodamol DA®.

### Example 15

### Preparation of a polysiloxane which corresponds in its statistical mean to the following formula:

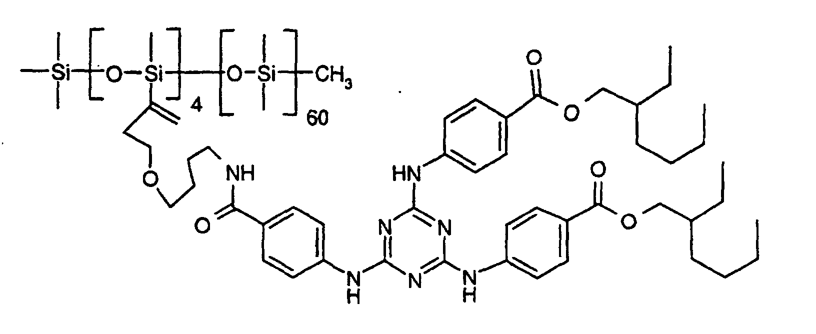

a) 4-Nitrobenzoic acid-3-propanolamide:
   The reaction described in example 14a was repeated using 2.5 equivalents of 3-amino propanol instead of propargyl amine and triethylamine. The reaction mixture was poured on water and seven times extracted with ethyl acetate. The combined ethyl acetate phases were dried with Na₂SO₄ and concentrated to yield 73% of a crystalline product, which was identified by NMR.
b) 4-Nitrobenzoic acid 3-propargyloxy propylamide:
   A 100 ml reaction flask was charged with 10 g of 4-nitrobenzoic acid 3-propargyloxy propylamide dissolved in 70 ml of THF and 5.25 g of Potassium tert. butoxyde and treated with 5.1 ml of propargyl bromide. After 50 min. the reaction mixture was heated to 60°C. for five hours and then distributed between water and ethyl acetate. The combined organic phases were concentrated and chromatographed in hexane / ethyl acetate =9:1 to yield 2.1 g of a yellow crystalline material, which again is identified by NMR.
c) 4-Aminobenzoic acid 3-propargyloxy propylamide:
   The reaction described in example 14b was repeated using 4-nitrobenzoic acid 3-propargyloxy propylamide instead of 4-nitrobenzoic acid 3-propargyloxy propylamide and heating only for 15 min. to 35°C. The liquid part of the reaction mixture was poured into water and extracted with CH₂Cl₂ until no product can be traced in the water phase. After concentration a yellow honey is obtained in 97% yield, the structure of which was proved by NMR. MS: 232(M⁺), 193, 120(100%).
d) 4-(4,6-Dichloro-(1,3,5)triazin-2-ylamino)-N-(3-propargyloxy-propyl-p-benzamide.
   The reaction described in example 14c was repeated using 4-aminobenzoic acid 3-propargyloxy propylamide instead of 4-aminobenzoic acid propargylamide. The product was filtered off to yield an off white powder in 65.5%. MS: 379(M⁺), 342, 340, 269, 267(100%).
e) 2,4-di-Anilino-p-carbo-(2'-ethyl-hexyloxy)-6-amino-{N-(3-propargyloxy-propyl)-p-benzamidyl}-1,3,5-triazine.
   The reaction described in example 14d was repeated using 4-(4,6-dichloro-(1,3,5)triazin-2-ylamino)-N-(3-propargyloxy-propyl-p-benzamide described above as starting material. The raw product was chromatographed in hexane / ethyl acetate =1:1. A yellowish powder was obtained in 85% yield. UV 306 nm (ε=104'380), m.p. 78-81°C.
f) Polysiloxane grafted triazine.
   A 25 ml reaction flask was charged with 0.4 g of the triazine received above in 10 ml of toluene, 0.55 g of polysiloxane Ae-151 of Wacker-Chemie GmbH. and a catalytic amount of divinyl-tetramethyl disiloxane platinum complex under inert atmosphere. The reaction mixture was heated to 100°C. for four days, washed with water/methanol = 1:10, concentrated and chromatographed in hexane ethyl acetate =7:3. 0.9 g of yellowish liquid was isolated, which is freely miscible with Cétiol LC® or Crodamol DA®. UV 306 nm (E=524).

### Example 16:

### Preparation of a O/W sunscreen lotion UV-B and UV-A:

Broad spectrum sunscreen lotion containing 2% of a compound of Ex. 1.

| Recipe: % | **compound** | **INCl name** |
|---|---|---|
| | | |

| **Part A:** | | |
|---|---|---|
| 2% | PARSOL MCX ™ | Octyl methoxycinnamate |
| 2% | Product of Example 1 | |
| 3% | PARSOL 1789 ™ | 4-tert.Butyl-4'methoxydibenzoyl methane |
| 12% | CETIOL LC® | Coco-caprylate/caprate |
| 4% | DERMOL 185® | Isostearyl neopentanoate |
| 0.25% | | Diethyleneglycolmonostearate |
| 1% | | Cetylalcohol |
| 0.25% | MPOB/PPOB® | Methyl-propylparabene |
| 0.1% | EDETA BD® | EDTA-disodium salt |
| 1% | AMPHISOL DEA ®(Giv.) | Diethanolamine cetylphosphate |

| **Part B:** | | |
|---|---|---|
| 20% | PERMULENE TR-1 ®(+%) | Acrylate C₁₀-C₃₀ Alkylacrylate-cross polymer |
| 48.6% | | water deion. |
| 5% | | 1,2-Propanediol |
| 0.8% | | Potassium hydroxide (10%) |

Part A was heated in a reactor to 85°C. Part B was slowly added within 10 min., followed by addition of KOH, cooling and degassing of the emulsion.

## Claims

1. Compounds of the general formula I, 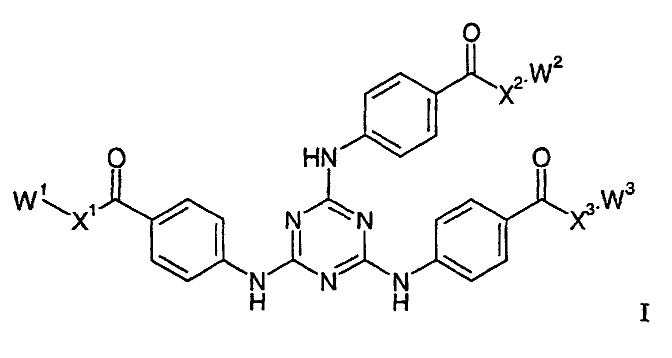 wherein
W¹, W² and W³ each independently signifies C₁-C₂₀ alkyl or a group SpSil;
X¹, X² and X³ each independently signifies O or NH;
Sp signifies a spacer group;
Sil signifies a silane, an oligosiloxane or a polysiloxane moiety;
with the proviso that at least one of W¹,W² and W³ signifies SpSil.

2. Compounds according to claim 1, 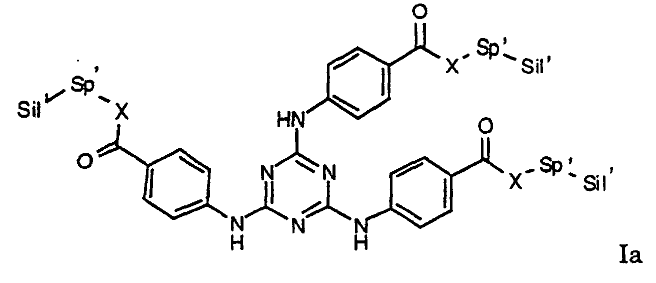 wherein
X signifies O or NH;
Sp' signifies a straight-chain or branched saturated or single or multiple unsaturated hydrocarbon group of 3 to 12 carbon atoms;
Sil' signifies the group SiR¹R²R³, wherein R¹, R² and R³ each independently signify C₁-C₆-alkyl, C₁-C₆-alkoxy or phenyl; or an oligosiloxane of the formula -SiMeₘ(OSiMe₃)ₙ, wherein Me is methyl and m is 0, or 2, n is 1, 2 or 3 and m+n are 3; or an oligosiloxane of the formulae A, A' and B 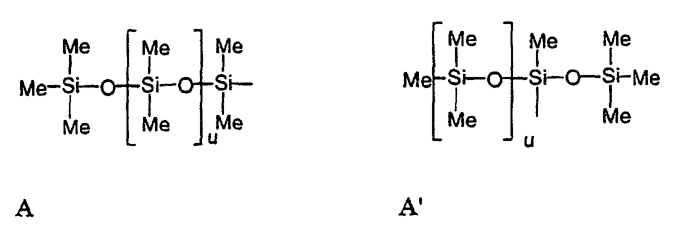 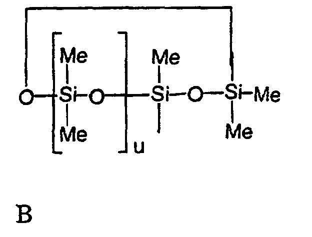
wherein Me is methyl and u is 0 to 6.

3. Compounds according to claim 1, wherein W¹, W² and W³ signify SpSil, or W¹ and W² signify C₁-C₂₀ alkyl and W³ signifies SpSil.

4. Compounds according to claim 1 or 3, wherein X¹, X² and X³ signify oxygen or X¹ and X² signify oxygen and X³ signifies NH.

5. Compounds according to any one of claims 1, 3 or 4, wherein the spacer is a C₃-C₁₂ divalent alkyl or alkylene chain optionally with one or several carbon atoms being replaced by oxygen atoms.

6. Compounds according to claim 5, wherein the spacer group signifies -(CH₂)₃-, -(CH₂)₄-, -(CH₂)₅-, -(CH)(CH₃)-(CH₂)-, -(CH₂)₂-CH=CH-, -C(=CH₂)-CH₂-, -C(=CH₂)-(CH₂)₂-O-(CH₂)₄-, (CH₂)₄-O-(CH₂)₂-.

7. Compounds according to any one of claims 1 to 5, wherein the spacer group signifies 3-propylene, 2-propylene, 2-propen-2-ylene, 2-propen-3-ylene, 2-methyl-3-propylene, 2-methyl-3-propenylene, 3-butylene, 4-butylene, 3-but-3-enylene, 4-but-3-enylene, 4-pentylene, 5-pentylene, 4-pent-4-enylene, 5-pent-4-enylene, 4- or 5-(3-methyl)penta-2,4-dienylene, 6-hexylene, 12-dodecylene or 11-dodecen-11-ylene.

8. Compounds according to any one of claims 1-7, wherein the silane-moiety signifies a group SiR¹R²R³, wherein R¹, R² and R³ each independently signify C₁-C₆ alkyl, C₁-C₆ alkoxy or phenyl.

9. Compounds according to claim 8, wherein the silane- moiety signifies -Si(CH₂-CH₃)₃- or -Si(CH₂-CH₂-CH₃)₃.

10. Compounds according to any one of claims 1, 3 to 6, wherein the oligosiloxane signifies a group of the general formula SiR¹⁰ₘ(OSiR¹⁰₃)ₙ with m = 0, 1 or 2; n = 3, 2 or 1 and m+n = 3; or a group of the general formula IIa, IIa' or IIb 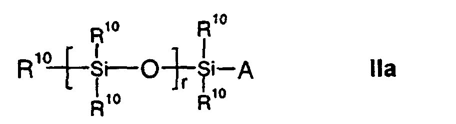 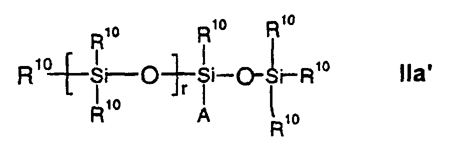 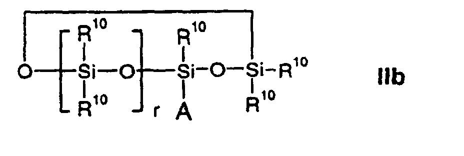 wherein
A signifies a bond to the spacer;
R¹⁰ signifies C₁-C₆ alkyl or phenyl;
r signifies 1 to 9, preferably 1 to 3.

11. Compounds according to any one of claims 1, 3 to 6, wherein the polysiloxane moiety signifies a group of the general formula IIIa or IIIb, 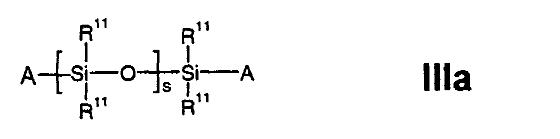 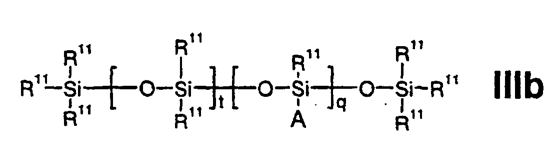 wherein
A is a bond to the spacer;
R¹¹ signifies C₁-C₆ alkyl or phenyl;
s has a value of from 4 to 250, preferably 5 to 150;
t has a value of from 5 to 250, preferably 5 to 150, more preferably a statistical mean of about 60;
q has a value of from 1 to 30, preferably 2 to 10, more preferably a statistical mean of about 4.

12. Compounds according to claim 10 or 11, wherein R¹⁰ and R¹¹ signify C₁-C₆ alkyl, more preferably C₁-C₄ alkyl, most preferably methyl.

13. Compounds according to claim 1, namely
2,4,6-tri-anilino-p-{carbo-4'-(1,1,3,3,3-pentamethyldisiloxanyl)-1'-butyloxy}-1,3,5-triazine,
2,4,6-tri-anilino-p-{carbo-4'-(1,1,2,3,5,5,5-heptamethyl trisiloxanyl)-1'-butyl-oxy}-1,3,5-triazine,
2,4,6-tri-anilino-p-{carbo-2'-methyl-3'-(1,1,3,3,3-pentamethyl disiloxanyl)-1'-propyloxy}-1,3,5-triazine,
2,4,6-tri-anilino-p-{carbo-5'-(1,1,3,3,3-pentamethyl disiloxanyl)-1'-pentyloxy}-1,3,5-triazine,
2,4,6-tri-anilino-p-{carbo-3'-(1,1,3,3,3-pentamethyl disiloxanyl)-1'-propyloxy}-1,3,5-triazine,
2,4,6-tri-anilino-p-{carbo-4'-(triethly silyl)-1'-but-3-enyloxy}-1,3,5-triazine and
2,4,6-tri-anilino-p-{carbo-4'-(triethyl silyl)-1'-butyloxy}-1,3,5-triazine.

14. Compounds according to claim 1, namely
2,4,6-tri-anilino-p-{carbo-7'-(triethyl silyl)-4'-oxa-heptyloxy}-1,3,5-triazine,
2,4,6-tri-anilino-p-{carbo-7'-(1,1,3,3,3-pentamethyl disiloxanyl)-4'-oxa-heptyloxy}-1,3,5-triazine,
2,4-di-anilino-p-carbo-(2'-ethyl-hexyloxy)-6-anilino-p-{carbo-7'-(1,1,3,3,3-pentamethyl disiloxanyl)-4'-oxa-heptyloxy}-1,3,5-triazine,
2,4-di-anilino-p-carbo-(2'-ethyl-hexyloxy)-6-anilino-p-{carbo-7'-(1,1,1,3,5,5,5-heptamethyl trisiloxanyl)-4'-oxa-heptyloxy}-1,3,5-triazine,
2,4-di-anilino-p-carbo-(2'-ethyl-hexyloxy)-6-amino{N-(-2'-(1,1,1,3,5,5,5-heptamethyltrisiloxanyl)-allyl)-p-benzamidyl}-1,3,5-triazine, or a polysiloxane which corresponds in its statistical mean to the following formula:

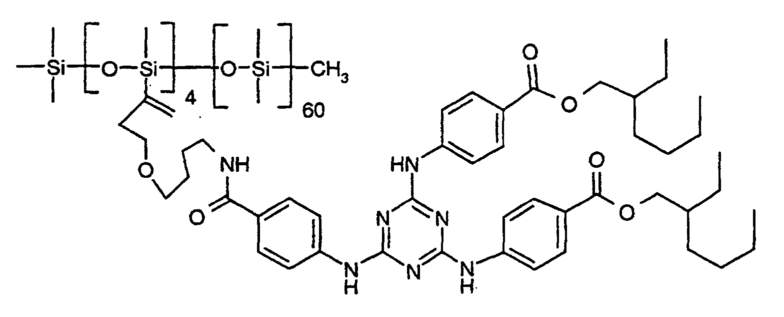

15. A lightscreening composition containing one or more compounds of any one of claims 1 to 14 in an amount from 0.1 to 20% by weight with respect to the composition, optionally further containing known UV-A or UV-B filters.

16. A process for the manufacture of the compounds of formula I according to claim 1, which process comprises
a) reacting a hydrosilane, an oligohydrosiloxane or a polyhydrosiloxane compound of the formula Sil-H, wherein Sil is as defined in claim 1, with a compound Sp''XH, wherein X is O or NH and Sp" has the same meaning as Sp except that it has one degree of unsaturation more than Sp at a temperature in the range of 0° to 200°C, preferably 40° to 110°C, in the presence of a metal catalyst, obtaining a compound of the formula SilSpXH,
b) reacting SilSpXH, the product of step a), with 4-aminobenzoic acid C₁-C₆ alkyl ester at a temperature in the range of 50° to 250°C in the presence of a basic-, acidic,- or a Lewis acid-catalyst, obtaining a compound of the formula A 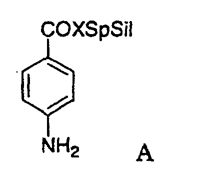 wherein X is O or NH, and Sp and Sil are as defined in claim 1,
c-1) treating cyanuric acid chloride with a compound of the formula A at a temperature in the range of 20° to 280°C, preferably of 50° to 150°C without or with an appropriate solvent, obtaining a compound of the formula I wherein W¹, W² and W³ signify a group SpSil, or
c-2) treating cyanuric acid chloride either at first with 4- aminobenzoic acid C₁-C₂₀ alkyl ester at a temperature in the range of 0° to 40°C, and then with a compound of the formula A above at a temperature in the range of 20° to 280°C, or treating cyanuric acid chloride at first with a compound of the formula A above at a temperature in the range of 0° to 40°C and then with 4-aminobenzoic C₁-C₂₀ alkyl ester at a temperature in the range of 20° to 280°C, obtaining a compound of the formula I wherein one or two of the groups W¹, W² and W³ signify C₁-C₂₀ alkyl and the remaining groups of W¹, W² and W³ signify SpSil.

17. The use of compounds of any one of claims 1 to 14 for the preparation of a light screening composition, especially for the preparation of a cosmetic composition useful for protecting human skin from sunlight radiation.

## Patentansprüche

1. Verbindungen der allgemeinen Formel I 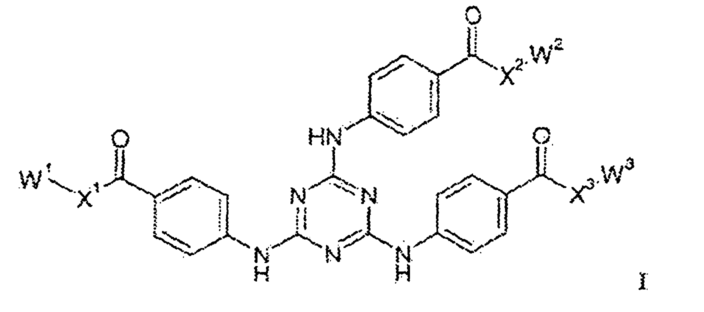 worin W¹, W² und W³ jeweils unabhängig C₁-C₂₀-Alkyl oder eine Gruppe SpSil bedeuten;
X¹, X² und X³ jeweils unabhängig O oder NH bedeuten;
Sp eine Spacergruppe bedeutet;
Sil ein Silan, ein Oligosiloxan oder eine Polysiloxaneinheit bedeutet;
mit dem Vorbehalt, dass mindestens einer der Reste W¹, W² und W³ SpSil bedeutet.

2. Verbindungen nach Anspruch 1 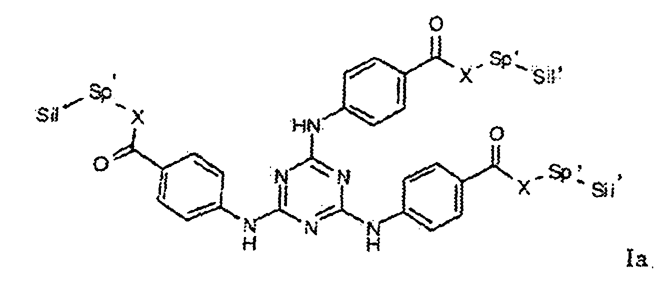 worin X O oder NH bedeutet;
Sp' eine geradkettige oder verzweigte gesättigte oder einfach oder mehrfach ungesättigte Kohlenwasserstoffgruppe mit 3 bis 12 Kohlenstoffatomen bedeutet;
Sil' die Gruppe SiR¹R²R³, worin R¹, R² und R³ jeweils unabhängig C₁-C₆-Alkyl, C₁-C₆-Alkoxy oder Phenyl bedeuten, oder ein Oligosiloxan der Formel -SiMeₘ(OSiMe₃)ₙ, worin Me Methyl ist und m 0, 1 oder 2 ist, n 1, 2 oder 3 ist und m+n 3 ist, oder ein Oligosiloxan gemäß einer der Formeln A, A' und B 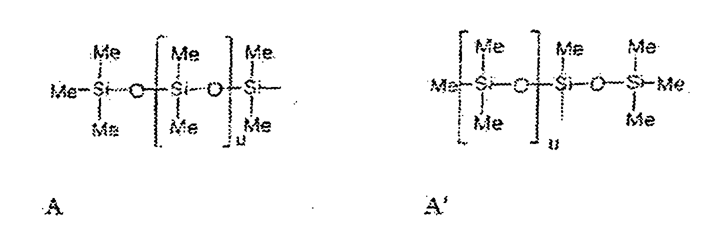 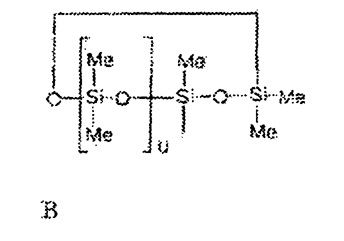 worin Me Methyl ist und u 0 bis 6 ist, bedeutet.

3. Verbindungen nach Anspruch 1, worin W¹, W² und W³ SpSil bedeuten oder W¹ und W² C₁-C₂₀-Alkyl bedeuten und W³ SpSil bedeutet.

4. Verbindungen nach Anspruch 1 oder 3, worin X¹, X² und X³ Sauerstoff bedeuten oder X¹ und X² Sauerstoff bedeuten und X³ NH bedeutet.

5. Verbindungen nach einem der Ansprüche 1, 3 oder 4, worin der Spacer eine divalente C₃-C₁₂-Alkyl- oder Alkylenkette ist, bei der gegebenenfalls ein oder mehrere Kohlenstoffatome durch Sauerstoffatome ersetzt wurden.

6. Verbindungen nach Anspruch 5, wobei die Spacergruppe -(CH₂)₃-, -(CH₂)₄-, -(CH₂)₅-, -(CH)(CH₃)-(CH₂)-, -(CH₂)₂-CH=CH-, -C(=CH₂)-CH₂-, -C(=CH₂)-(CH₂)₂-O-(CH₂)₄-, (CH₂)₄-O-(CH₂)₂- bedeutet.

7. Verbindungen nach einem der Ansprüche 1 bis 5, worin die Spacergruppe 3-Propylen, 2-Propylen, 2-Propen-2-ylen, 2-Propen-3-ylen, 2-Methyl-3-propylen, 2-Methyl-3-propenylen, 3-Butylen, 4-Butylen, 3-But-3-enylen, 4-But-3-enylen, 4-Pentylen, 5-Pentylen, 4-Pent-4-enylen, 5-Pent-4-enylen, 4- oder 5-(3-Methyl)penta-2,4-dienylen, 6-Hexylen, 12-Dodecylen oder 11-Dodecen-11-ylen bedeutet.

8. Verbindungen nach einem der Ansprüche 1 bis 7, worin die Silaneinheit eine Gruppe SiR¹R²R³ bedeutet, worin R¹, R² und R³ jeweils unabhängig C₁-C₆-Alkyl, C₁-C₆-Alkoxy oder Phenyl bedeuten.

9. Verbindungen nach Anspruch 8, worin die Silaneinheit -Si(CH₂-CH₃)₃- oder -Si (CH₂-CH₂-CH₃)₃ bedeutet.

10. Verbindungen nach einem der Ansprüche 1, 3 bis 6, worin das Oligosiloxan eine Gruppe der allgemeinen Formel SiR¹⁰ₘ(OSiR¹⁰₃)ₙ bedeutet, mit m = 0, 1 oder 2; n = 3, 2 oder 1 und m+n = 3 oder eine Gruppe der allgemeinen Formel IIa, IIa' oder IIb 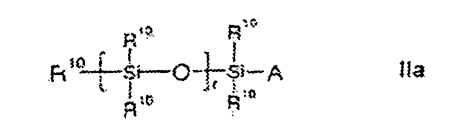 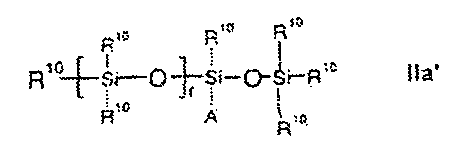 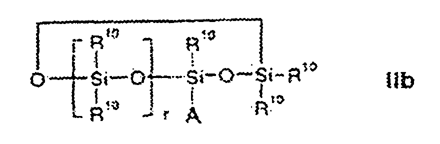 worin
A eine Bindung an den Spacer bedeutet;
R¹⁰ C₁-C₆-Alkyl oder Phenyl bedeutet;
r 1 bis 9, bevorzugt 1 bis 3 bedeutet.

11. Verbindungen nach einem der Ansprüche 1, 3 bis 6, worin die Polysiloxaneinheit eine Gruppe der allgemeinen Formeln IIIa oder IIIb bedeutet 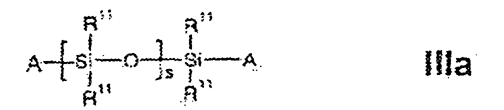 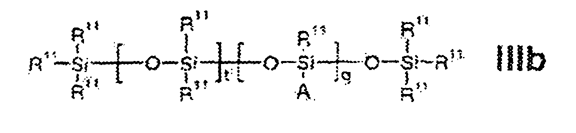 worin
A eine Bindung an den Spacer ist;
R¹¹ C₁-C₆-Alkyl oder Phenyl bedeutet;
s einen Wert von 4 bis 250, bevorzugt 5 bis 150 hat;
t einen Wert von 5 bis 250, bevorzugt 5 bis 150, bevorzugter einen statistischen Mittelwert von etwa 60 hat;
q einen Wert von 1 bis 30, bevorzugt 2 bis 10, bevorzugter einen statistischen Mittelwert von etwa 4 hat.

12. Verbindungen nach Anspruch 10 oder 11, worin R¹⁰ und R¹¹ C₁-C₆-Alkyl, bevorzugter C₁-C₄-Alkyl, am meisten bevorzugt Methyl bedeuten.

13. Verbindungen nach Anspruch 1, nämlich
2,4,6-Trianilino-p-{carbo-4'-(1,1,3,3,3-pentamethyldisiloxanyl)-1'-butyloxy}-1,3,5-triazin,
2,4,6-Trianilino-p-{carbo-4'-(1,1,1,3,5,5,5-heptamethyltrisiloxanyl)-1'-butyloxy}-1,3,5-triazin,
2,4,6-Trianilino-p-{carbo-2'-methyl-3'-(1,1,3,3,3-pentamethyldisiloxanyl)-1'-propyloxy}-1,3,5-triazin,
2,4,6-Trianilino-p-{carbo-5'-(1,1,3,3,3-pentamethyldisiloxanyl)-1'-pentyloxy}-1,3,5-triazin,
2,4,6-Trianilino-p-{carbo-3'-(1,1,3,3,3-pentamethyldisiloxanyl)-1'-propyloxy}-1,3,5-triazin,
2,4,6-Trianilino-p-{carbo-4'-(triethylsilyl)-1'-but-3-enyloxy}-1,3,5-triazin und
2,4,6-Trianilino-p-{carbo-4'-(triethylsilyl)-1'-butyloxy}-1,3,5-triazin.

14. Verbindungen nach Anspruch 1, nämlich
2,4,6-Trianilino-p-{carbo-7'-(triethylsilyl)-4'-oxaheptyloxy}-1,3,5-triazin,
2,4,6-Trianilino-p-{carbo-7'-(1,1,3,3,3-pentamethyldisiloxanyl)-4'-oxaheptyloxy}-1,3,5-triazin,
2,4-Dianilino-p-carbo-(2'-ethylhexyloxy)-6-anilino-p-{carbo-7'-(1,1,3,3,3-pentamethyldisiloxanyl)-4'-oxaheptyloxy}-1,3,5-triazin,
2,4-Dianilino-p-carbo-(2'-ethylhexyloxy)-6-anilino-p-{carbo-7'-(1,1,1,3,5,5,5-heptamethyltrisiloxanyl)-4'-oxaheptyloxy}-1,3,5-triazin,
2,4-Dianilino-p-carbo-(2'-ethylhexyloxy)-6-amino{N-(2'-(1,1,1,3,5,5,5-heptamethyltrisiloxanyl)allyl)-p-benzamidyl}-1,3,5-triazin oder
ein Polysiloxan, das im statistischen Mittel der folgenden Formel entspricht:

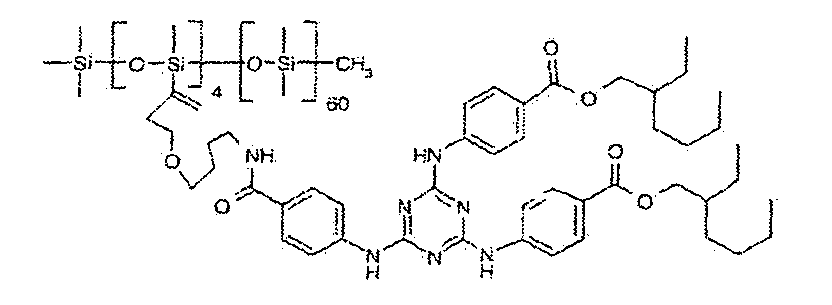

15. Lichtschutzzusammensetzung enthaltend eine oder mehrere Verbindungen nach einem der Ansprüche 1 bis 14 in einer Menge von 0,1 bis 20 Gew.-% bezogen auf die Zusammensetzung, die gegebenenfalls weiterhin bekannte UV-A- oder UV-B-Filter enthält.

16. Verfahren zur Herstellung der Verbindungen der Formel I nach Anspruch 1, wobei das Verfahren umfasst:
a) dass eine Hydrosilan-, Oligohydrosiloxan- oder Polyhydrosiloxanverbindung der Formel Sil-H, worin Sil wie in Anspruch 1 definiert ist, mit einer Verbindung Sp"XH, worin X O oder NH ist und Sp" die gleiche Bedeutung wie Sp hat, außer dass es einen Grad an Ungesättigtheit hat, der um eins höher ist als bei Sp bei einer Temperatur im Bereich von 0 bis 200°C, bevorzugt 40 bis 110°C in Gegenwart eines Metallkatalysators umgesetzt wird, wobei eine Verbindung der Formel SilSpXH erhalten wird,
b) SilSpXH, das Produkt von Stufe a), mit 4-Aminobenzoesäure-C₁-C₆-alkylester bei einer Temperatur im Bereich von 50 bis 250°C in Gegenwart eines basischen, sauren oder Lewis-Säure-Katalysators umgesetzt wird, wobei eine Verbindung der Formel A 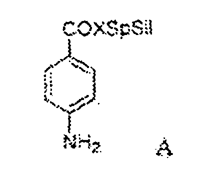 erhalten wird, worin X O oder NH ist und Sp und Sil wie in Anspruch 1 definiert sind,
c-1) Cyanursäurechlorid mit einer Verbindung der Formel A bei einer Temperatur von 20 bis 280°C, bevorzugt 50 bis 150°C mit oder ohne geeignetes Lösungsmittel behandelt wird, wobei eine Verbindung der Formel I erhalten wird, wobei W¹, W² und W³ eine Gruppe SpSil bedeuten, oder
c-2) Cyanursäurechlorid entweder zuerst mit 4-Aminobenzoesäure-C₁-C₂₀-alkylester bei einer Temperatur im Bereich von 0 bis 40°C und dann mit einer Verbindung der Formel A oben bei einer Temperatur im Bereich von 20 bis 280°C behandelt wird oder Cyanursäurechlorid zuerst mit einer Verbindung der Formel A oben bei einer Temperatur im Bereich von 0 bis 40°C und dann mit 4-Aminobenzoesäure-C₁-C₂₀-alkylester bei einer Temperatur im Bereich von 20 bis 280°C behandelt wird, wobei eine Verbindung der Formel I erhalten wird, worin ein oder zwei der Gruppen W¹, W² und W³ C₁-C₂₀-Alkyl bedeuten und die verbleibenden Gruppen von W¹, W² und W³ SpSil bedeuten.

17. Verwendung von Verbindungen nach einem der Ansprüche 1 bis 14 zur Herstellung einer Lichtschutzzusammensetzung insbesondere zur Herstellung einer kosmetischen Zusammensetzung, die geeignet ist, menschliche Haut vor Sonneneinstrahlung zu schützen.

## Revendications

1. Composés de la formule générale I : 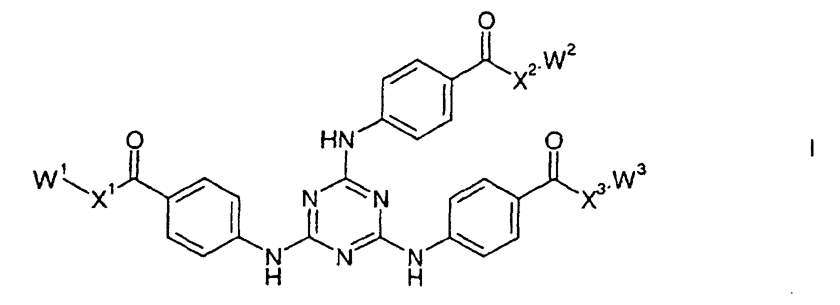 dans laquelle :
W¹, W² et W³ représentent chacun indépendamment un groupe alkyle en C₁-C₂₀ ou un groupe SpSil;
X¹, X² et X³ représentent chacun indépendamment O ou NH;
Sp représente un groupe d'espacement;
Sil représente un fragment de silane, d'oligosiloxane ou de polysiloxane;
à la condition qu'au moins un des W¹, W² et W³ représente SpSil.

2. Composés suivant la revendication 1, 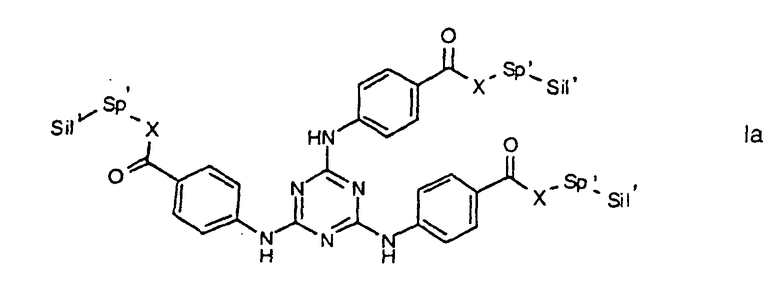 dans laquelle :
X représente O ou NH;
Sp' représente un groupe hydrocarboné insaturé simple ou multiple ou saturé à chaîne droite ou ramifié de 3 à 12 atomes de carbone;
Sil' représente le groupe SiR¹R²R³, dans lequel R¹, R² et R³ représentent chacun indépendamment un groupe alkyle en C₁-C₆, alcoxy en C₁-C₆ ou phényle; ou un oligosiloxane de la formule -SiMeₘ(OSiMe₃)ₙ, dans laquelle Me est du méthyle et m est égal à 0, 1 ou 2, n est égal à 1, 2 ou 3 et m+n valent 3; ou un oligosiloxane des formules A, A' et B : 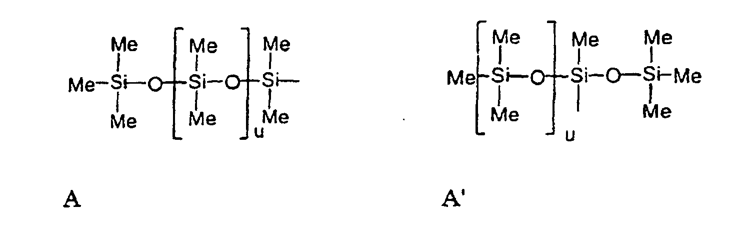 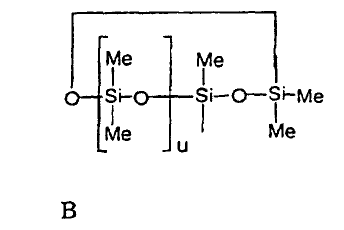
dans lesquelles Me est du méthyle et u vaut de 0 à 6.

3. Composés suivant la revendication 1, dans lesquels W¹, W² et W³ représentent SpSil, ou bien W¹ et W² représentent un groupe alkyle en C₁-C₂₀ et W³ représente SpSil.

4. Composés suivant l'une ou l'autre des revendications 1 et 3, dans lesquels X¹, X² et X³ représentent de l'oxygène ou bien X¹ et X² représentent de l'oxygène et X³ représente NH.

5. Composés suivant l'une quelconque des revendications 1, 3 et 4, dans lesquels le groupe d'espacement est une chaîne alkyle ou alkylène divalente en C₃-C₁₂ éventuellement avec un ou plusieurs atomes de carbone remplacés par des atomes d'oxygène.

6. Composés suivant la revendication 5, dans lesquels le groupe d'espacement représente un groupe (CH₂)₃-, -(CH₂)₄-, -(CH₂)₅-, -(CH)(CH₃)-(CH₂)-, -(CH₂)₂-CH=CH-, -C(=CH₂)-CH₂-, -C(=CH₂)-(CH₂)₂-O-(CH₂)₄-, (CH₂)₄-O-(CH₂)₂-.

7. Composés suivant l'une quelconque des revendications 1 à 5, dans lesquels le groupe d'espacement est du 3-propylène, 2-propylène, 2-propén-2-ylène, 2-propén-3-ylène, 2-méthyl-3-propylène, 2-méthyl-3-propénylène, 3-butylène, 4-butylène, 3-but-3-énylène, 4-but-3-énylène, 4-pentylène, 5-pentylène, 4-pent-4-énylène, 5-pent-4-énylène, 4-ou 5-(3-méthyl)penta-2,4-diénylène, 6-hexylène, 12-dodécylène ou 11-dodécén-11-ylène.

8. Composés suivant l'une quelconque des revendications 1 à 7, dans lesquels le fragment de silane représente un groupe SiR¹R²R³, dans lequel R¹, R² et R³ représentent chacun indépendamment un groupe alkyle en C₁-C₆, alcoxy en C₁-C₆ ou phényle.

9. Composés suivant la revendication 8, dans lesquels le fragment de silane représente un groupe -Si(CH₂-CH₃)₃- ou -Si(CH₂-CH₂-CH₃)₃.

10. Composés suivant l'une quelconque des revendications 1, 3 à 6, dans lesquels l'oligosiloxane représente un groupe de la formule générale SiR¹⁰ₘ(OSiR¹⁰₃)ₙ avec m=0, 1 ou 2, n=3, 2 ou 1 et m+n=3, ou un groupe de la formule générale IIA, IIa' ou IIb : 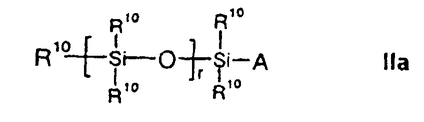 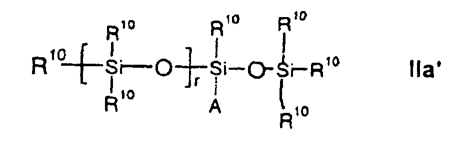 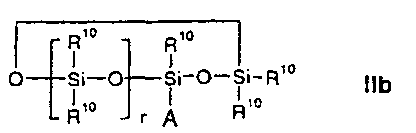 dans lesquelles :
A représente une liaison au groupe d'espacement;
R¹⁰ représente un groupe alkyle en C₁-C₆ ou phényle;
r vaut de 1 à 9, avantageusement 1 à 3.

11. Composés suivant l'une quelconque des revendications 1, 3 à 6, dans lesquels le fragment de polysiloxane représente un groupe de la formule générale Illa ou Illb : 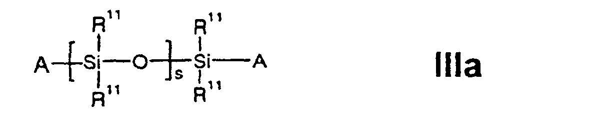 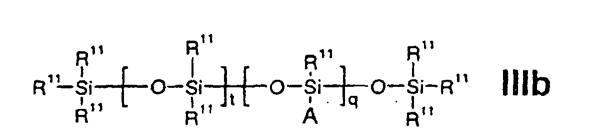 dans lesquelles :
A est une liaison au groupe d'espacement;
R¹¹ représente un groupe alkyle en C₁-C₆ ou phényle;
s a une valeur de 4 à 250, avantageusement de 5 à 150.
t a une valeur de 5 à 250, avantageusement de 5 à 150, le plus avantageusement une moyenne statistique d'environ 60;
q a une valeur de 1 à 30, avantageusement de 2 à 10, plus avantageusement une moyenne statistique d'environ 4.

12. Composés suivant l'une ou l'autre des revendications 10 et 11, dans lesquels R¹⁰ et R¹¹ représentent un groupe alkyle en C₁-C₆, plus avantageusement un groupe alkyle en C₁-C₄, le plus avantageusement du méthyle.

13. Composés suivant la revendication 1, à savoir :
la 2,4,6-trianilino-p-{carbo-4'-(1,1,3,3,3-pentaméthyldisiloxanyl)-1'-butyl-oxy}-1,3,5-triazine,
la 2,4,6-trianilino-p-{carbo-4'-(1,1,1,3,5,5,5-heptaméthyl trisiloxanyl)-1'-butyloxy}-1,3,5-triazine,
la 2,4,6-trianilino-p-{carbo-2'-méthyl-3'-(1,1,3,3,3-pentaméthyl disiloxanyl)-1'-propyloxy}-1,3,5-triazine,
la 2,4,6-trianilino-p-{carbo-5'-(1,1,3,3,3-pentaméthyl disiloxanyl)-1'-pentyl-oxy}-1,3,5-triazine,
la 2,4,6-trianilino-p-(carbo-3'-(1,1,3,3,3-pentaméthyl disiloxanyl)-1'-propyloxy}-1,3,5-triazine,
la 2,4,6-trianilino-p-{carbo-4'-(triéthyl silyl)-1'-but-3-ényloxy}-1,3,5-triazine, et
la 2,4,6-trianilino-p-{carbo-4'-(triéthyl silyl)-1'-butyloxy}-1,3,5-triazine.

14. Composés suivant la revendication 1, à savoir :
la 2,4,6-trianilino-p-{carbo-7'-(triéthyl silyl)-4'-oxaheptyloxy}-1,3,5-triazine,
la 2,4,6-trianilino-p-{carbo-7'-(1,1,3,3,3-pentaméthyl disiloxanyl)-4'-oxaheptyloxy}-1,3,5-triazine,
la 2,4-dianilino-p-carbo-(2'-éthylhexyloxy)-6-anilino-p-{carbo-7'-(1,1,3,3,3-pentaméthyl disiloxanyl)-4'-oxaheptyloxy}-1,3,5-triazine,
la 2,4-dianilino-p-carbo-(2'-éthylhexyloxy)-6-anilino-p-{carbo-7'-(1,1,1,3,5,5,5-heptaméthyl trisiloxanyl)-4'-oxaheptyloxy}-1,3,5-triazine,
la 2,4-dianilino-p-carbo-(2'-éthylhexyloxy)-6-amino-{N-(2'-(1,1,1,3,5,5,5-heptaméthyl trisiloxanyl)allyl)-p-benzamidyl}-1,3,5-triazine, ou un polysiloxane qui correspond dans sa moyenne statistique à la formule suivante :

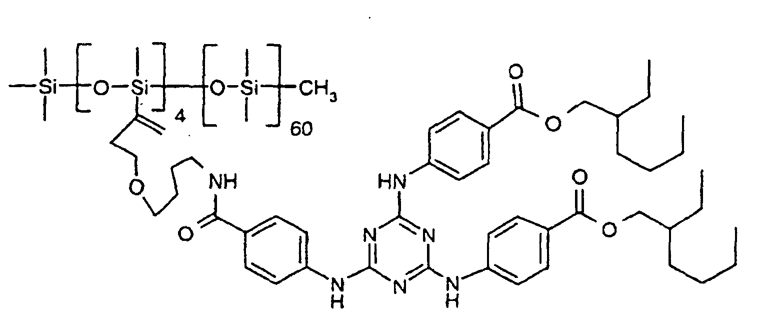

15. Composition de protection contre la lumière contenant un ou plusieurs composés suivant l'une quelconque des revendications 1 à 14, en une quantité de 0,1 à 20% en poids de la composition, éventuellement contenant de plus des filtres UV-A ou UV-B connus.

16. Procédé de fabrication des composés de formule I suivant la revendication 1, lequel procédé comprend :
a) la réaction d'un composé d'hydrosilane, d'oligohydrosiloxane ou de polyhydrosiloxane de la formule Sil-H, dans laquelle Sil est tel que défini à la revendication 1, avec un composé Sp"XH, dans lequel X est O ou NH et Sp" a la même signification que Sp à l'exception qu'il a un degré d'insaturation de plus que Sp à une température allant de 0° à 200°C, avantageusement de 40° à 110°C, en présence d'un catalyseur métallique, pour obtenir un composé de la formule SilSpXH,
b) la réaction de SilSpXH, le produit de l'étape a), avec un ester alkylique en C₁-C₆ d'acide 4-aminobenzoïque à une température allant de 50° à 250°C en présence d'un catalyseur basique, acide ou d'acide de Lewis, pour obtenir un composé de la formule A : 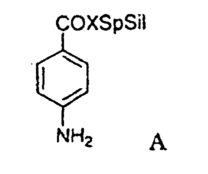
dans laquelle X est O ou NH, et Sp et Sil sont tels que définis à la revendication 1,
c-1) le traitement de chlorure d'acide cyanurique avec un composé de la formule A à une température allant de 20° à 280°C, avantageusement de 50° à 150°C sans ou avec un solvant approprié, pour obtenir un composé de la formule I dans laquelle W¹, W² et W³ représentent un groupe SpSil, ou bien,
c-2) le traitement de chlorure d'acide cyanurique soit d'abord avec un ester alkylique en C₁-C₂₀ d'acide 4-aminobenzoïque à une température allant de 0° à 40°C, et ensuite avec un composé de la formule A ci-dessus à une température allant de 20° à 280°C, ou le traitement de chlorure d'acide cyanurique d'abord avec un composé de la formule A ci-dessus à une température allant de 0° à 40°C et ensuite avec un ester alkylique en C₁-C₂₀ d'acide 4-aminobenzoïque à une température allant de 20° à 280°C, pour obtenir un composé de la formule I dans laquelle un ou deux des groupes W¹, W² et W³ représentent un groupe alkyle en C₁-C₂₀ et les groupes restants parmi W¹, W² et W³ représentent SpSil.

17. Utilisation de composés suivant l'une quelconque des revendications 1 à 14, pour la préparation d'une composition de protection contre la lumière, en particulier pour la préparation d'une composition cosmétique utilisable pour la protection de la peau humaine contre le rayonnement de la lumière solaire.
